# FASCICULE DE BREVET EUROPEEN

(11) **EP 0 522 964 B1**
(45) Date de publication et mention de la délivrance du brevet: **04.10.2000**
(21) Numéro de dépôt: 92401980.5
(22) Date de dépôt: 09.07.1992
(51) Int. Cl.: C07D 239/48, C07D 239/50, A61K 7/06

(54) **Compositions pour freiner la chute des cheveux et pour induire et stimuler leur croissance à base de dérivés de 2,4-diamino pyrimidine 3-oxyde**
Zusammenstellungen, um den Haarausfall zu hemmen und um Haarwuchs zu induzieren und zu stimulieren auf Basis von 2,4-Diamino-pyrimidin-3-Oxydderivaten
Compositions to arrest hair loss and to induce and stimulate hair growth based on 2,4-diamino-pyrimidin-3-oxide derivatives

(30) Priorité: 11.07.1991 FR 9108764
(43) Date de publication de la demande: 13.01.1993
(73) Titulaire: L'OREAL, 75008 Paris (FR)
(72) Inventeur: Terranova, Eric, F-92600 Asnières (FR); Galey, Jean-Baptiste, F-75005 Paris (FR); Hocquaux, Michel, F-75012 Paris (FR); Maignan, Jean, F-93290 Tremblay-les-Gonesse (FR); Tuloup, Rémy, F-35190 Miniac-sous-Bécherel (FR)
(74) Mandataire: Casalonga, Axel

(56) Documents cités:
- EP-A- 0 356 271
- DE-A- 1 695 959
- US-A- 4 139 619
- CHEMICAL ABSTRACTS, , no. 92, 1980, Columbus, Ohio, US; abstract no. 128848Z, W. COWDEB ET AL.: 'PYRIMIDINE N-OXIDE II' page 696 ;colonne 2 ;
- : "", J. DERMATOL., E. NOWAK, 1985, , , Vol. 24, no. , pages 82 à 87
- : "", AUST. J. CHEM., 1981, , , Vol. 34, no. 9, pages 1921 à 1933

## Description

La présente invention est relative à de nouvelles compositions pour freiner la chute des cheveux et pour induire et stimuler leur croissance à base de dérivés de 2,4-diamino pyrimidine 3-oxyde, ainsi que les nouveaux composés utilisés dans ces compositions.

On connaît déjà dans l'état de la technique, le 2,4-diamino 6-piperidino pyrimidine 3-oxyde ou "Minoxidil" pour ses propriétés anti-hypertensives mais également pour son utilisation dans le traitement de la chute des cheveux, de la pelade, de la dermatite desquamante, de l'alopécie.

Le document EP-A-0 356 271 décrit des nouveaux dérivés de diamino-2,4 pyrimidine oxyde-3 et leur utilisation pour le traitement et la prévention de la chute des cheveux.

Le document DE-A-1 695 959 décrit un procédé de préparation de 1,2-dihydro-1-hydroxypyrimidine.

Le document US-A-4 139 619 concerne l'utilisation pour stimuler la croissance des cheveux du 6-amino-1,2-dihydro-1-hydroxy-2 imino-4-piperidinopyrimidine, plus connu sous le nom de minoxidil.

La demanderesse a découvert de nouvelles compositions pour le traitement et la prévention de la chute des cheveux, utilisées notamment en application topique, contenant une famille particulière de composés 6- alcoxy ou 6-thioalkyl 2,4-diamino pyrimidine 3-oxyde pouvait être substitués en position 5.

Les composés retenus par la demanderesse sont de manière surprenante très efficaces pour la repousse des cheveux et notamment pour induire et stimuler leur croissance. En outre, ils présentent une remarquable solubilité dans des milieux habituellement utilisés en cosmétique ou pharmacie.

L'invention a donc pour objet, de nouvelles compositions destinées au traitement et à la prévention de la chute des cheveux contenant des composés particuliers 6-alcoxy ou 6-thioalkyl 2,4-diamino pyrimidine 3-oxyde éventuellement substitués en position 5.

L'invention a également pour objet de nouveaux composés utilisés dans ces compositions.

Un autre objet de l'invention est l'utilisation de ces composés particuliers pour la préparation d'un médicament destiné au traitement thérapeutique de la chute des cheveux. D'autres objets apparaîtront à la lecture de la description et des exemples qui suivent.

Les compositions conformes à l'invention sont essentiellement caractérisées par le fait qu'elles contiennent dans un milieu physiologiquement acceptable au moins un composé répondant à la formule (I) : 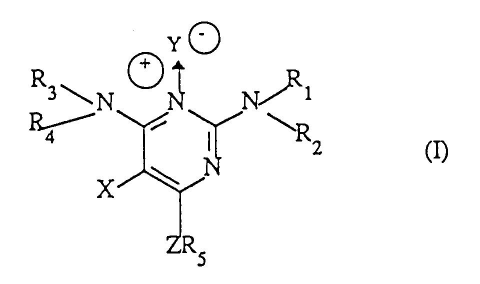 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle, un radical arylalkyle, un radical hydroxyalkyle ou aminoalkyle dans lequel le radical alkyle possède 1 à 6 atomes de carbone ;
X désigne un atome d'hydrogène, un atome d'halogène;
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃;
   ainsi que ses sels d'addition d'acides physiologiquement acceptables, sous réserve que le composé de formule (I) sort différent du 2,4 diamino-6 hydroxy 5 bromopyrimidine et du 2,4 diamino 6 thiophènylpyrimidine 3-oxi.

Les atomes d'halogène dans la structure de formule (I) sont de préférence le chlore, le brome, le fluor ou l'iode.

Les radicaux alkyle en C₁-C₆ sont choisis de préférence parmi méthyle, éthyle, propyle, n-butyle, n-pentyle, n-héxyle.

Les radicaux alkyle en C₁-C₁₂ sont choisis de préférence parmi méthyle, éthyle, propyle, éthyl-2 hexyle, décyle, octyle, dodécyle.

Les groupements alcényle en C₃-C₁₂ sont choisis de préférence parmi allyle, n-butényle, hexényle, dodécényle.

Les radicaux aryle ou aralkyle sont choisis de préférence parmi phényle, benzyle, tolyle.

Les composés de formule (I) conformes à l'invention peuvent être transformés en leurs sels d'additions d'acides pharmaceutiquement ou cosmétiquement acceptables tels que les sels des acides sulfurique, chlorhydrique, bromhydrique, phosphorique, acétique, benzoïque, salicylique, glycolique, succinique, tartrique, nicotinique, maléique, pamoïque, méthane sulfonique, picrique, lactique, etc...

Les composés de formule générale (I) sont nouveaux à l'exception du compose 2,4-diamino 6-hydroxy 5-bromopyrimidine 3-oxyde décrit dans le document W.L.F. ARMAREGO et P.WARING, Aust. J. Chem., 1981, 34 (9), 1921-33 et du composé 2,4-diamino 6-thiophényl pyrimidine 3-oxyde décrit dans le document W. B. COWDEN ET N. W. JACOBSEN, Aust. J. Chem 1979, 32 (9), 2049-57.

Les composés conformes à la présente invention peuvent être obtenus à partir de composés dérivés de 2,4-diamino pyrimidine 3-oxyde de formule : 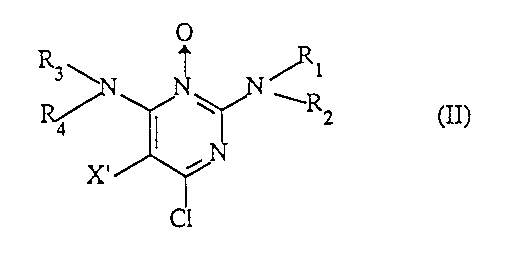 dans laquelle :
R₁ et R₃ désignent un hydrogène et R₂ et R₄ désignent un hydrogène ou un alkyle en C₁-C₄ et X′ désigne un hydrogène.

Les composés particuliers de départ de formule (IIA) suivante: 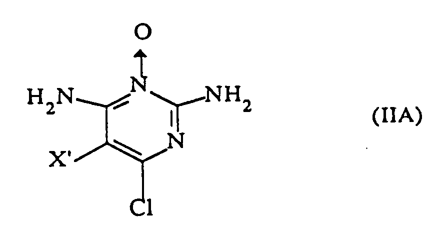 sont obtenus par cyclisation de la guanidine (1) avec un cyanoacétate d'alkyle de formule (2), suivie d'une halogénation en position 6 du dérivé 2,4-diamino pyrimidine de formule (3) obtenu. On effectue ensuite une oxydation en position 3 du noyau pyrimidine, de préférence dans un solvant protique polaire en présence d'un péracide carboxylique R′CO₃H où R′ désigne un radical alkyle ou aryle. Ce procédé peut être représenté par le schéma suivant :

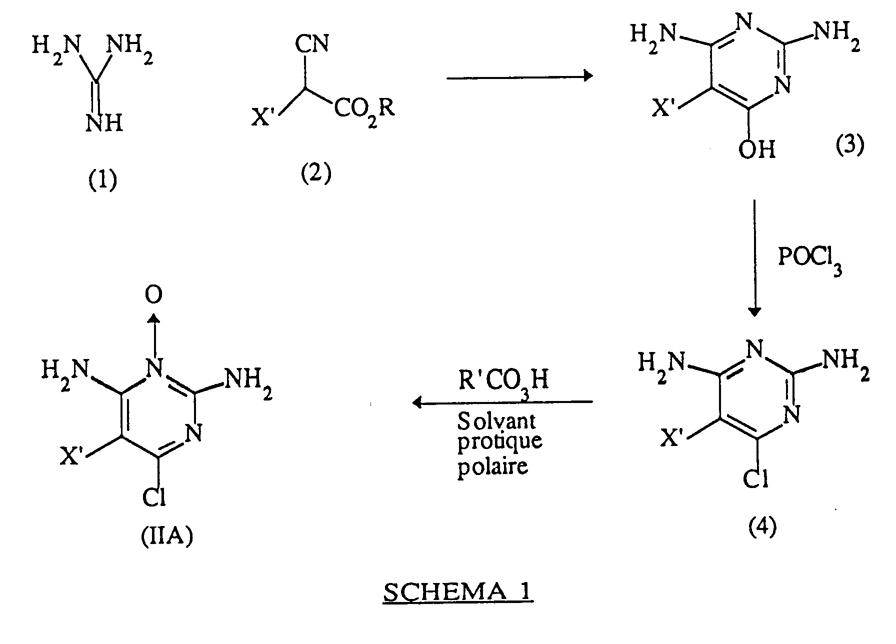

Les composés de départ particuliers de formule (IIB) suivante: 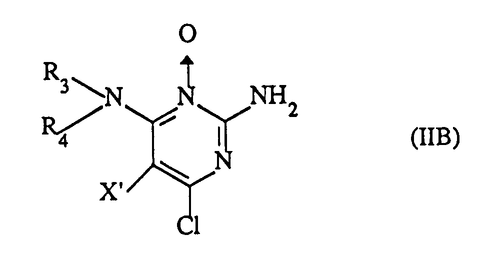 avec R₃=H et différent de R₄, peuvent être obtenus dans un premier temps par cyclisation de la guanidine avec le malonate de diéthyle monosubstitué ou non en alpha, suivie d'une réaction de chloration sur le dérivé pyrimidine ainsi obtenu. On introduit ensuite l'amine NHR₃R₄ par substitution nucléophile aromatique sur le noyau pyrimidine du produit obtenu puis on effectue une oxydation en position 3, selon le principe décrit précédemment. Ce procédé peut être représenté par le schéma suivant :

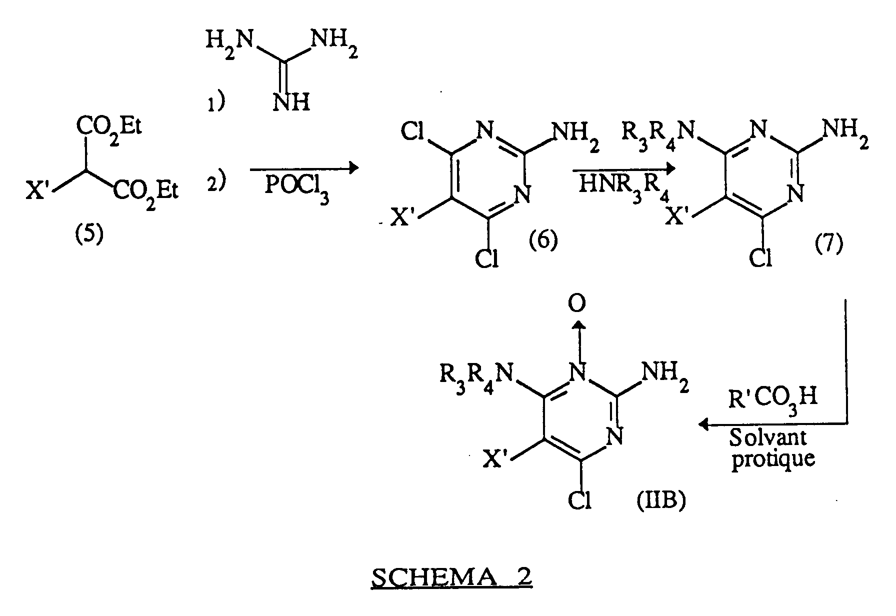

Les composés particuliers de départ de formule (IIC) : 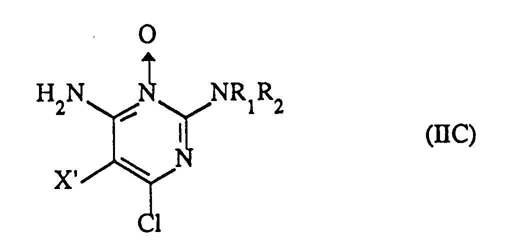 dans laquelle R₁ désigne hydrogène et R₂ désigne alkyle, sont obtenus par oxydation au moyen d'un péracide du dérivé de pyrimidine de formule : 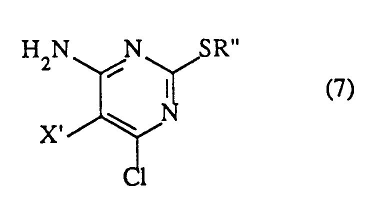 où R'' désigne un aryle ou un alkyle, suivie d'une substitution nucléophile aromatique du groupement -SO₂R'' obtenu par l'amine NHR₁R₂ sur le noyau pyrimidine. On effectue ensuite une oxydation en position 3 sur le noyau pyrimidine dans les mêmes conditions que précédemment.

Les composés de départ particuliers de formule suivante: 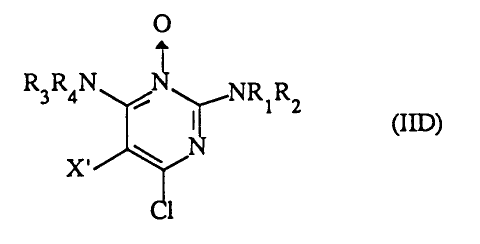 dans laquelle R₁ et R₃ désignent hydrogène et R₂ et R₄ désignent alkyle, peuvent être obtenus selon le schéma réactionnel suivant :

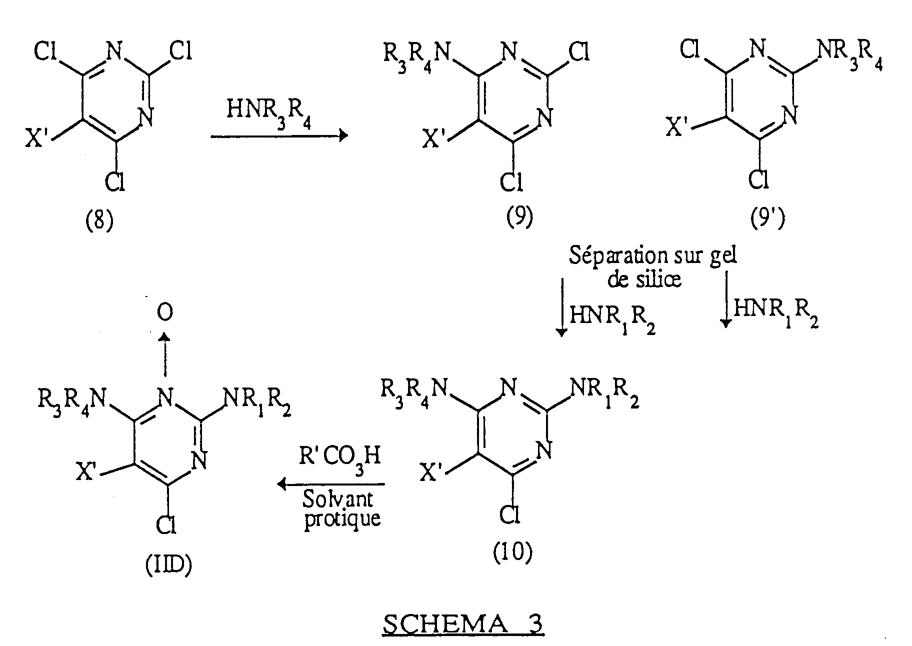

On introduit successivement, à partir du composé de formule (8), deux équivalents de l'amine NHR₃R₄ sur le noyau pyrimidine, puis après séparation sur gel de silice des produits formés (9) et (9′), on introduit la deuxième amine NHR₁R₂.

On effectue ensuite une oxydation en position 3 du noyau pyrimidine comme précédemment.

Les composés de la présente invention particuliers de formule suivante : 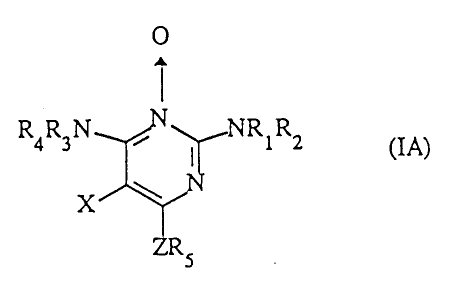 où R₁, R₂, R₃, R₄, Z et R₅ ont les mêmes significations indiquées ci-dessus dans la formule générale (I), et X désigne l'hydrogène, peuvent être obtenus en faisant réagir les composés de formule (II), soit avec un alcoolate ⁻OR₅, W⁺ dans l'alcool correspondant R₅OH, où W désigne un métal alcalin tel que sodium, potassium, lithium, soit avec un thiolate ⁻SR₅, W⁺ en présence d'un solvant tel que le diméthylformamide.

Ce procédé peut être représenté par le schéma réactionnel suivant :

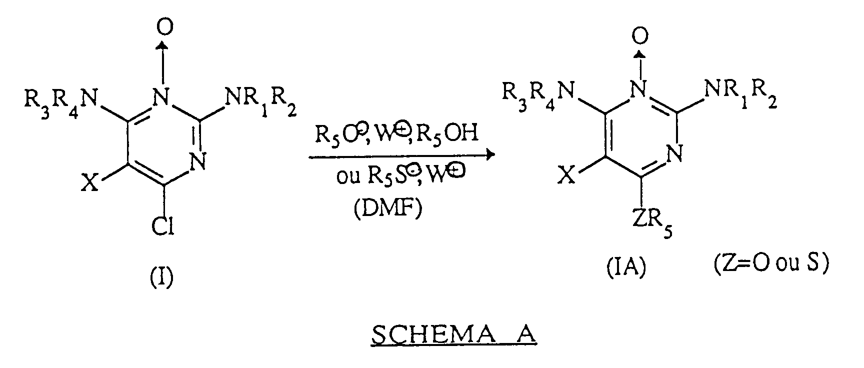

Les composés particuliers de l'invention de formule suivante : 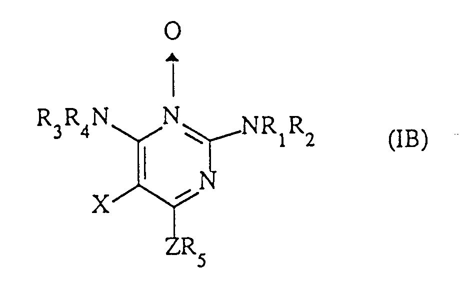 où R₁, R₂, R₃, R₄, R₅, Z ont les mêmes significations indiquées ci-dessus et X désigne un atome d'halogène, peuvent être obtenus en faisant réagir un composé de formule (II) dans laquelle X′ désigne l'hydrogène avec un agent halogénant. Dans le cas où X désigne le chlore, l'iode ou le brome, on utilise par exemple un N-halogéno succinimide de formule (III) : 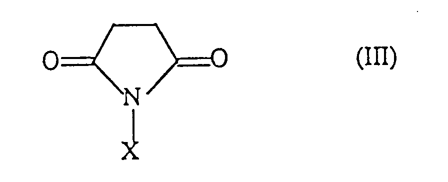 en présence d'un alcool tel que le méthanol.

On substitue ensuite l'atome de chlore en position 6 du noyau pyrimidine par le groupe -ZR₅ selon le procédé décrit ci-dessus dans le schéma A.

Le procédé peut être représenté par le schéma suivant : 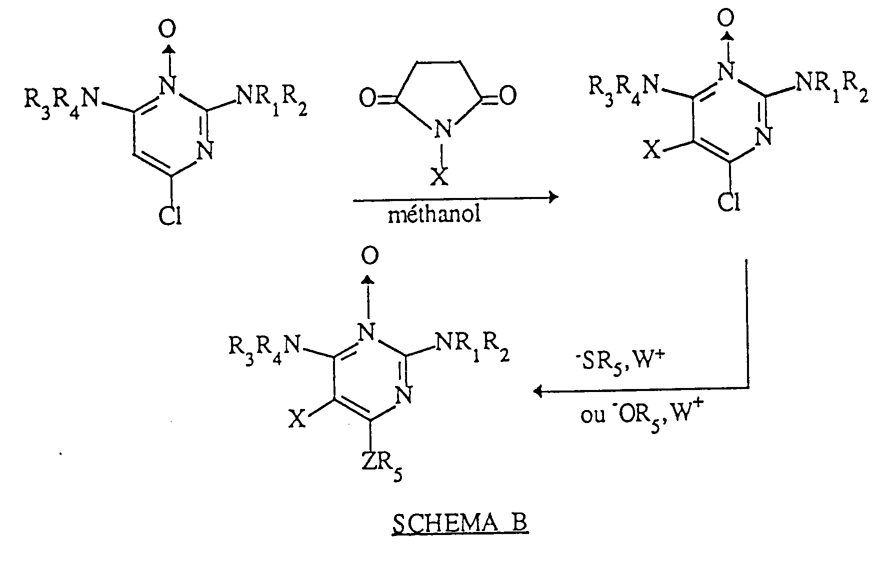 Les composés particuliers de l'invention de formule générale (I) dans laquelle Y désigne un atome d'oxygène, peuvent être transformés en leurs homologues O-sulfates de formule (IF) définie ci-après par sulfatation chimique selon les méthodes classiques décrites dans la littéraire (J. Med. Chem., 1983, 26, pages 1791-1793).

On utilise comme réactif de sulfatation les complexes de trioxyde de soufre-pyridine, de trioxyde de soufre-triéthylamine ou de trioxyde de soufre-éthyldiisopropylamine.

Les solvants utilisés sont de préférence le diméthylformamide, le chloroforme, l'acétonitrile ou leurs mélanges binaires.

La température est de l'ordre de 0 à 25°C et le temps de réaction varie de 1 à 24 heures.

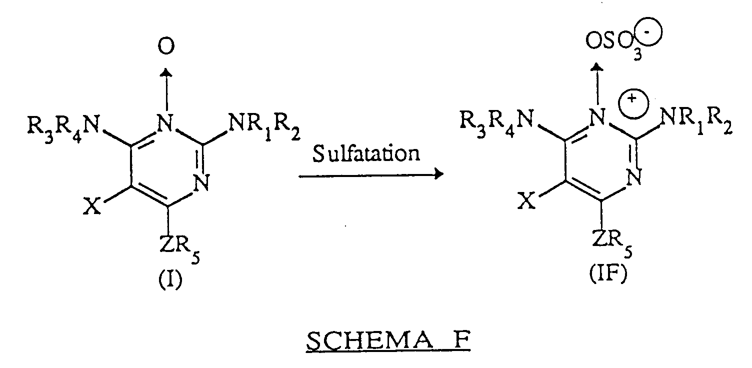

Parmi les composés de l'invention de formule générale (I) dans laquelle Z désigne l'oxygène, les composés particulièrement préférés sont ceux pour lesquels R₁, R₂, R₃, R₄ désignent hydrogène, X désigne le clore ou le groupement nitro et R₅ désigne n-butyle.

Parmi les composés de l'invention de formule (I) dans laquelle Z désigne le soufre, les composés particulièrement préférés sont ceux pour lesquels X désigne le chlore, R₁, R₂, R₃ et R₄ désignent un atome d'hydrogène, et R₅ désigne méthyle, éthyle, n-butyle, 2-hydroxyéthyle, 2-aminoéthyle ou phényle.

Les compositions conformes à la présente invention, contenant dans un milieu physiologiquement acceptable, au moins un composé répondant à la formule (I) ou un de ses sels d'addition d'acides physiologiquement acceptables, peuvent être appliquées dans le domaine cosmétique ou pharmaceutique, notamment en application topique. Elles sont destinées pour le traitement et la prévention de la chute des cheveux et notamment de la pelade, de l'alopécie ainsi que des dermatites desquamantes et la stimulation de leur repousse.

Ces compositions peuvent comporter, à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées en pharmacie se présentent sous forme d'onguent, de teinture, de crème, de pommade, de poudre, de timbre, de tampon imbibé, de solution, d'émulsion ou de dispersion vésiculaire, de lotion, de gel, de spray ou de suspension. Elles peuvent être, soit anhydres, soit aqueuses, selon l'indication clinique.

Les composés selon l'invention sont présents dans ces compositions pharmaceutiques à des concentrations comprises entre 0,1 et 10% en poids, et en particulier comprises entre 0,2 et 5% en poids.

Les compositions cosmétiques sont notamment destinées à être utilisées sous forme de lotion, de gel, de savon, de shampooing, d'aérosol ou de mousse et contiennent, dans un support cosmétiquement acceptable, au moins un composé de formule (I) ou l'un de ses sels d'addition d'acides.

La concentration de ces composés de formule (I) dans ces compositions est, de préférence, comprise entre 0,01 et 5% en poids et en particulier entre 0,05 et 3% en poids.

Les compositions conformes à l'invention peuvent contenir différents additifs habituellement utilisés en cosmétique ou en pharmacie et en particulier des substances actives, telles que des agents hydratants comme la thiamorpholine et ses dérivés ou l'urée; des agents antiséborrhéiques, tels que la S-carboxyméthylcystéine, la S-benzylcystéamine, et leurs dérivés; la thioxolone.

Les composés conformes à l'invention peuvent être associés à des composés améliorant encore leur activité sur la repousse et/ou sur le freinage de la chute des cheveux, tels que les composés suivants :
- les esters d'acide nicotinique, dont plus particulièrement les nicotinates d'alkyle en C₁-C₆ et notamment le nicotinate de méthyle ou le nicotinate de benzyle;
- les agents anti-inflammatoires stéroïdiens et non stéroïdiens bien connus dans l'état de la technique et en particulier l'hydrocortisone, ses sels et ses dérivés, l'acide niflumique;
- les rétinoïdes et plus particulièrement l'acide t-trans rétinoïque appelé encore trétinoïne, l'isotrétinoïne, le rétinol ou la vitamine A et ses dérivés, tels que l'acétate, le palmitate ou le propionate, le motrétinide, l'étrétinate, le t-trans rétinoate de zinc;
- les agents antibactériens choisis plus particulièrement parmi les macrolides, les pyranosides et les tétracyclines et notamment l'érythromycine;
- les agents antagonistes de calcium, tels que plus particulièrement la Cinnarizine et le Diltiazem;
- des hormones, telles que l'estriol ou des analogues ou la thyroxine et ses sels;
- des agents antiandrogènes, tels que l'oxendolone, la spironolactone, le diéthylstilbestrol;
- des capteurs de radicaux OH, tels que le diméthylsulfoxyde.

On peut également associer avec les composés de l'invention, éventuellement en mélange avec les autres, des composés tels que le Diazoxyde correspondant au méthyl-3 chloro-7 [2H] benzothiadiazine 1,2,4-dioxyde-1,1; la Spiroxasone ou 7-(acétylthio)-4′,5′-dihydrospiro [androst 4-ène- 17,2′-(3′H)furan] -3 one; des phospholipides, tels que la lécithine; les acides linoléique et linolénique; l'acide salicylique et ses dérivés décrits dans le brevet français 2 581 542, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters, des lactones et leurs sels correspondants; l'anthraline ou la trihydroxy-1,8,9 anthracène, les caroténoïdes, les acides eicosatétraynoïque-5,8,11,14 ou eicosatriynoïque-5,8,11, leurs esters et amides.

Les composés conformes à l'invention peuvent également être associés à des agents tensio-actifs dont notamment ceux choisis parmi les agents tensio-actifs non ioniques et amphotères.

Parmi les tensio-actifs non ioniques, on citera les polyhydroxy propyléthers décrits notamment dans les brevets français n° 1 477 048; 2 091 516; 2 169 787; 2 328 763; 2 574 786; les alkyl(C₈-C₉)phénols oxyéthylénés comportant de 1 à 100 moles d'oxyde d'éthylène et de préférence 5 à 35 moles d'oxyde d'éthylène; les alkylpolyglycosides de formule :

CₙH₂ₙ₊₁ (C₆H₁₀O₅)ₓH (A)

dans laquelle n varie de 8 à 15 inclus et x de 1 à 10 inclus.

Parmi les agents tensio-actifs amphotères, on citera plus particulièrement les amphocarboxyglycinates et les amphocarboxy propionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléthers d'alkylèneglycols, en particulier d'alkyle en C₁-C₄, d'alkylène en C₁-C₄ glycols ou de dialkylèneglycols, de préférence de dialkylène en C₁-C₄ glycols, d'alcool éthylique et d'eau, le solvant glycolique désignant plus particulièrement le monoéthyléther de l'éthylèneglycol, le mono méthyléther du propylèneglycol, le monométhyléther du diéthylène glycol.

Les composés conformes à l'invention peuvent également être introduits dans des supports gélifiés ou épaissis, tels que des supports essentiellement aqueux gélifiés par des hétérobiopolysaccharides, comme la gomme de xanthane, les scléroglucanes ou les dérivés de cellulose, des supports hydroalcooliques gélifiés par des polyhydroxyéthylacrylates ou méthacrylates ou des supports essentiellement aqueux épaissis en particulier par des acides polyacryliques réticulés par un agent polyfonctionnel, tel que les CARBOPOL vendus par la Société GOODRICH.

Ces compositions peuvent également contenir des agents conservateurs, des agents stabilisants, des agents régulateurs de pH, des agents modificateurs de pression osmotique, des agents émulsifiants, des filtres UVA et UVB, des agents antioxydants, tels que l'α-tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique et choisis plus particulièrement parmi les alcools inférieurs en C₁-C₄, comme l'alcool éthylique, l'alcool isopropylique, l'alcool tertiobutylique, les alkylèneglycols, les alkyléthers d'alkylèneglycol et de dialkylèneglycol, tels que le monoéthyléther d'éthylèneglycol, le monométhyléther de propylène glycol, le monométhyléther de diéthylèneglycol. Les solvants, lorsqu'ils sont présents, le sont dans des proportions comprises entre 1 et 80% en poids par rapport au poids total de la composition.

Les épaississants sont présents de préférence dans des proportions comprises entre 0,1 et 5% en poids et en particulier entre 0,4 et 3% en poids par rapport au poids total de la composition.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Un autre objet de l'invention est constitué par l'utilisation de la composition contenant les composés de formule (I) définie ci-dessus, pour la préparation d'un médicament ayant pour effet d'induire ou de stimuler la croissance des cheveux et de freiner leur chute.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

Les compositions peuvent notamment être utilisées dans le traitement de la pelade, de la chute des cheveux, des dermatites desquamantes.

Les exemples suivants sont destinés à illustrer l'invention sans pour autant présenter un caractère limitatif.

### EXEMPLES DE PREPARATION

### EXEMPLE 1

### PREPARATION DE 2,4-DIAMINO 5-CHLORO 6-n-BUTYLOXY PYRIMIDINE 3-OXYDE.

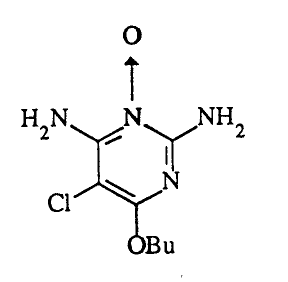

### a) Préparation de 2,4-diamino 5,6-dichloropyrimidine.

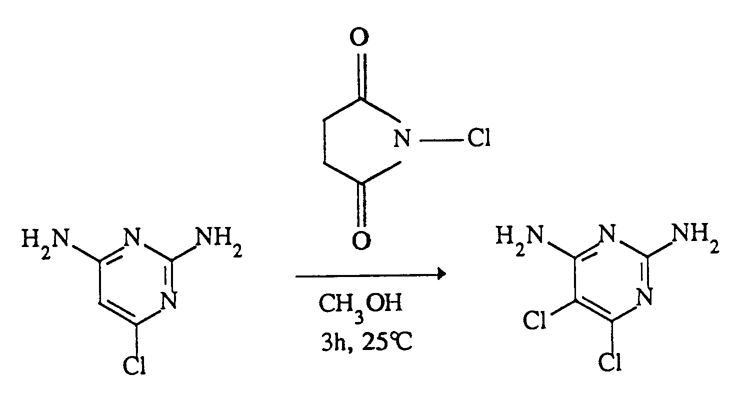

A une solution de 45 g de 2,4-diamino 6-chloropyrimidine dans le méthanol (1030 ml), on additionne par portions 50 g de N-chloro succinimide à 23°C. Après complète addition, on agite le milieu réactionnel à température ambiante pendant 3 heures. On évapore le méthanol sous pression réduite et on reprend le brut obtenu au reflux de 850 ml d'eau pendant 1 heure. Après filtration, le produit brut est recristallisé deux fois dans un mélange EtOH:H₂O=(80:20) pour donner 44,5 g de 2,4-diamino 5,6-dichloropyrimidine.
Rendement = 80%.
PF_{K} = 218°C.

| Analyse élémentaire pour C₄H₄N₄Cl₂ ; M = 179. | | | | |
|---|---|---|---|---|
| | C | H | N | Cl |
| Calculé | 26,82 | 2,23 | 31,28 | 39,66 |
| Trouvé | 26,87 | 2,22 | 31,27 | 39,62 |

Les spectres de RMN ¹H et de masse sont en accord avec la structure attendue.

### b) Préparation de 2,4-diamino 5-chloro 6-n-butyloxy pyrimidine.

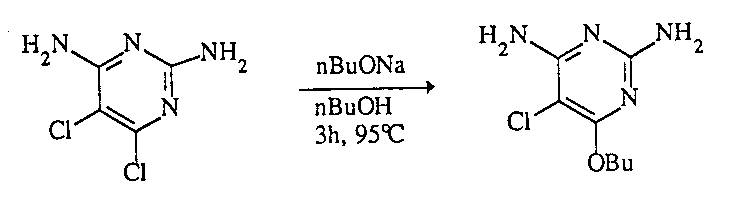

On additionne 15,6 g de sodium dans 1,3 l de n-butanol.

Après dissolution complète du sodium, on ajoute 87 g de 2,4-diamino 5,6-dichloro pyrimidine.

On chauffe à 95°C pendant 3 heures.

Après refroidissement du mélange, on ajoute 300 ml d'eau, agite et décante.

La phase organique est mise à sec.

On recristallise le produit obtenu dans 1 l de mélange d'eau/ méthanol (1/1).

On obtient 95,7 g de produit blanc.
Rendement : 91%.
PF_{K} = 135°C.

| Analyse élémentaire pour C₈H₁₃N₄OCl 0,1 H₂O ; M = 218,3. | | | | | |
|---|---|---|---|---|---|
| | C | H | N | O | Cl |
| Calculé | 43,98 | 6,05 | 25,65 | 8,06 | 16,26 |
| Trouvé | 43,90 | 6,05 | 25,68 | 8,17 | 16,20 |

Les spectres de RMN ¹H et de masse sont conformes à la structure attendue.

### c) Préparation dc 2,4-diamino 5-chloro 6-butyloxy pyrimidine 3-oxyde.

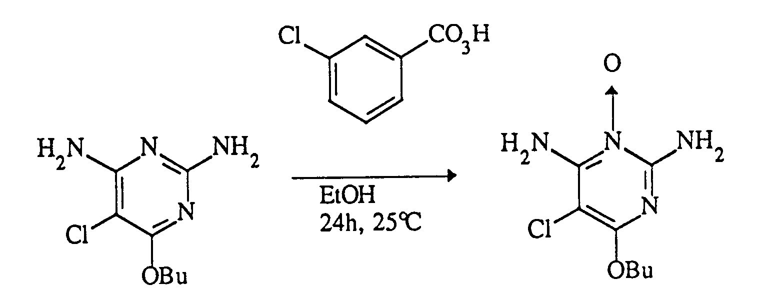

On additionne 50 g de 2,4-diamino 5-chloro 6-butyloxy pyrimidine dans 1,5 l d'éthanol absolu.

La solution est refroidie à 5°C, puis on ajoute peu à peu 145 g d'acide m-chloro perbenzoïque (à 55%).

On laisse remonter à température ambiante et agite pendant 24 heures.

Après mise à sec du mélange réactionnel, on ajoute 400 ml d'eau et acidifie par 70 ml d'acide chlorhydrique concentré.

On filtre le précipité obtenu.

Les eaux-mères sont basifiées par 90 ml de soude, le précipité obtenu est filtré puis lavé à l'eau.

Après recristallisation dans 300 ml d'un mélange eau/éthanol (1/1) et lavage à l'acétone, on obtient 15 g.
Rendement = 28%.
PF_{K} = 140°C.

| Analyse élémentaire pour C₈H₁₃N₄O₂Cl ; M = 232,5. | | | | | |
|---|---|---|---|---|---|
| | C | H | N | O | Cl |
| Calculé | 41,29 | 5,59 | 24,09 | 13,76 | 15,27 |
| Trouvé | 41,35 | 5,60 | 23,87 | 14,00 | 15,22 |

Les spectres de RMN ¹H et de masse sont en accord avec la structure attendue.

### EXEMPLE 2

### Préparation de 2,4-diamino 6-thiophényl pyrimidine 3-oxyde.

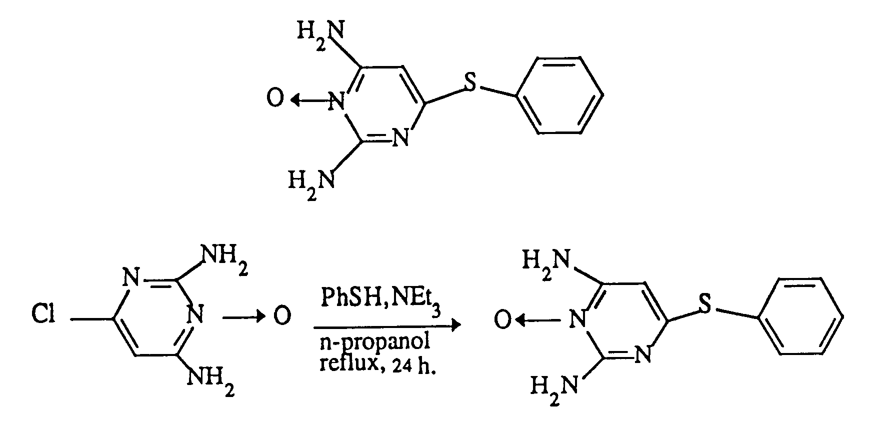

On place 5 g de 2,4-diamino 6-chloro pyrimidine 3-oxyde, 6,85 g de thiophénol, 11,5 g de triéthylamine dans 110 ml de n-propanol. Le milieu réactionnel est chauffé au reflux pendant 24 heures. On évapore à sec. Le résidu est repris dans 25 ml d'éther éthylique, agité 1/2 heure, filtré sur verre fritté, rincé par 2x25 ml d'éther éthylique. On reprend le précipité dans 75 ml d'eau. Le pH est ajusté à 1 avec de l'acide chlorhydrique concentré. Après 1/2 heure d'agitation, on revient à pH8 par addition de soude concentrée. Le précipité est filtré, rincé par 10 ml d'eau, puis recristallisé deux fois dans un mélange éthanol/eau 75/25. On obtient 3,35 g de 2,4-diamino 6-thiophényl pyrimidine 3-oxyde.
Rendement = 46%.
Point de fusion = décomposition à partir de 265°C.

| Analyse élémentaire pour C₁₀H₁₀N₄OS ; M = 234. | | | | | |
|---|---|---|---|---|---|
| | C | H | N | O | S |
| Calculé | 51,28 | 4,27 | 23,93 | 6,84 | 13,68 |
| Trouvé | 51,24 | 4,34 | 24,06 | 7,03 | 13,58 |

Les spectres de RMN ¹H et de masse sont en accord avec la structure attendue.

### EXEMPLE 3

### Préparation de 2,4-diamino 6-thioéthyl pyrimidine 3-oxyde.

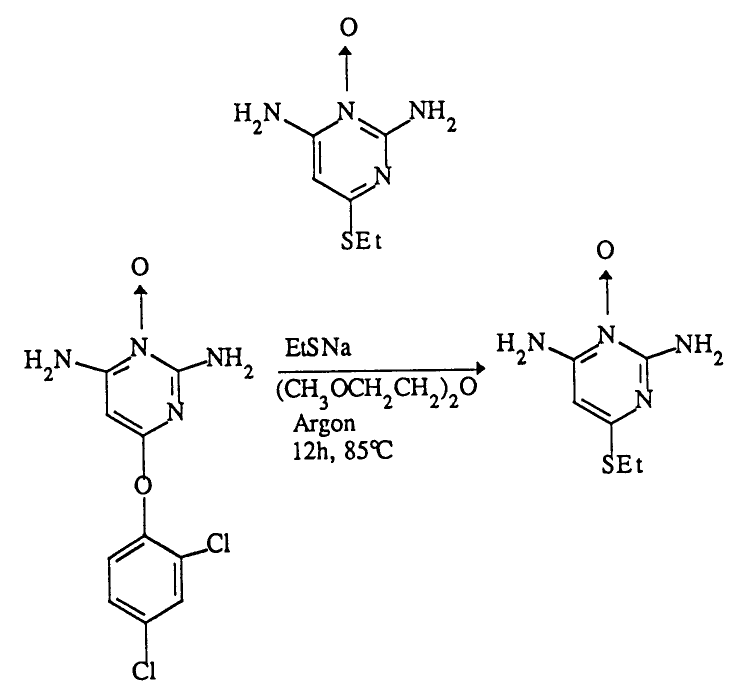

### Mode opératoire :

On dissout 30 g de 2,4-diamino 6-(2′,4′-dichlorophénoxy) pyrimidine 3-oxyde dans 400 ml de diglyme à 85°C, puis on additionne par portions 23 g de thiolate de sodium. On agite la réaction à 85°C sous argon pendant 12 heures. On évapore le solvant et on reprend le brut dans une solution d'éthanol chlorhydrique jusqu'à obtenir un pH de 2. On filtre le précipité (précipité 1) et on concentre le filtrat qu'on reprend dans l'éther diéthylique. On obtient un solide que l'on joint au précipité 1. On dissout le tout dans l'eau et on amène le pH à 8 à l'aide d'une solution de soude. On filtre le précipité et on l'agite pendant 30 minutes dans l'éther diéthylique. Après filtration, on obtient 9,6 g de 2,4-diamino 6-thioéthyl pyrimidine 3-oxyde.
Rendement = 50%.

| Analyse élémentaire pour C₆H₁₀N₄OS ; M = 186. | | | | | |
|---|---|---|---|---|---|
| | C | H | N | O | S |
| Calculé | 38,70 | 5,37 | 30,10 | 8,60 | 17,20 |
| Trouvé | 38,75 | 5,39 | 30,02 | 8,72 | 17,14 |

Les spectres de RMN ¹H et de masse sont en accord avec la structure attendue.

### EXEMPLE 4

### Préparation de 2,4-diamino 6-thiobutyl pyrimidine 3-oxyde.

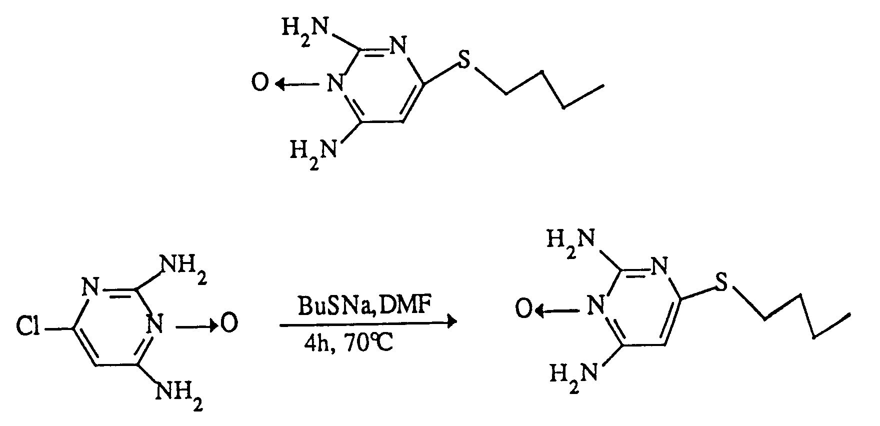

On dissout 1 g de 2,4-diamino 6-chloro pyrimidine 3-oxyde dans 50 ml de diméthylformamide à 70°C. On ajoute 1,75 g de butanethiolate de sodium. Le milieu réactionnel est agité pendant 4 heures à 70°C. On évapore à sec. Le résidu est repris dans 10 ml d'eau. Le précipité obtenu est filtré sur verre fritté, rincé par 5 ml d'eau. On recristallise dans 8 ml de méthanol. Le précipité est rincé par 10 ml d'éther éthylique. On obtient 720 mg de 2,4-diamino 6-thiobutyl pyrimidine 3-oxyde.
Rendement = 57%.
Point de fusion = 178°C.

| Analyse élémentaire pour C₈H₁₄N₄OS ; M = 214. | | | | | |
|---|---|---|---|---|---|
| | C | H | N | O | S |
| Calculé | 44,86 | 6,54 | 26,17 | 7,47 | 14,95 |
| Trouvé | 44,74 | 6,50 | 26,25 | 7,60 | 15,01 |

Les spectres de RMN ¹H et de masse sont en accord avec la structure attendue.

### EXEMPLE 5

### Préparation de 2,4-diamino 5-chloro 6-thiobutyl pyrimidine 3-oxyde.

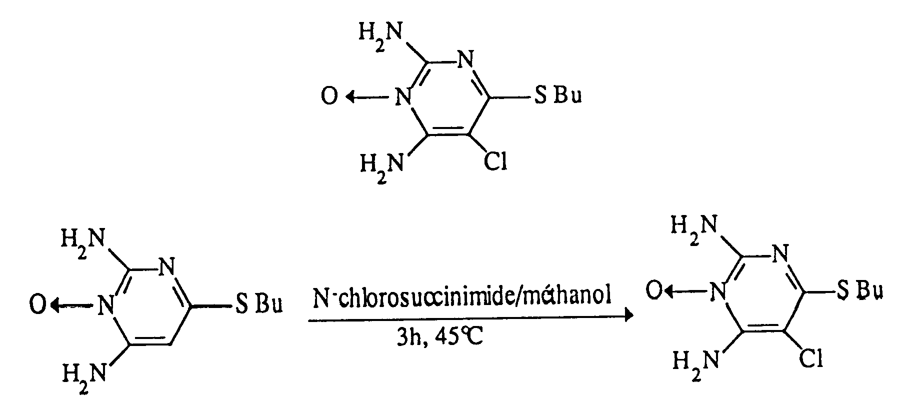

On dissout 1 g de 2,4-diamino 6-thiobutyl pyrimidine 3-oxyde dans 100 ml de méthanol. On ajoute 935 mg de N-chlorosuccinimide. Le milieu réactionnel est agité à 45°C pendant 3 heures puis évaporé à sec. Le résidu est repris dans 2 ml d'éthanol chlorhydrique (8,6M). On ajoute 100 ml d'éther éthylique. Le précipité blanc obtenu est filtré sur verre fritté et rincé par 10 ml d'éther éthylique. On reprend le précipité dans 5 ml d'eau. Le pH est ajusté à 8 par addition de soude 10N. Après 1/2 heure d'agitation, le précipité est filtré sur verre fritté, rincé par 2x5 ml d'eau et séché sous vide sur anhydride phosphorique. On recristallise dans 10 ml d'éthanol-eau 50-50. On obtient 290 mg de 2,4-diamino 5-chloro 6-thiobutyl pyrimidine 3-oxyde.
Rendement = 25%.
Point de fusion = décomposition à partir de 70°C.

| Analyse élémentaire pour C₈H₁₃Cl N₄OS ; M = 248,5. | | | | | | |
|---|---|---|---|---|---|---|
| | C | H | Cl | N | O | S |
| Calculé | 38,63 | 5,23 | 14,28 | 22,53 | 6,44 | 12,88 |
| Trouvé | 38,53 | 5,26 | 14,18 | 22,31 | 6,61 | 12,80 |

Les spectres de RMN ¹³C et de masse sont en accord avec la structure attendue.

### EXEMPLE 8

### Préparation de 2,4-diamino 6-thiométhyl pyrimidine 3-oxyde.

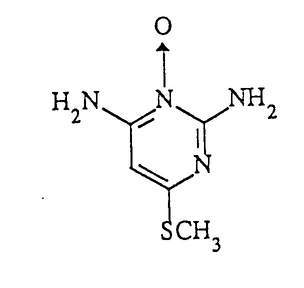

### Mode opératoire :

Dans une solution de 70 cm³ d'éthanol absolu, contenant une pastille de soude et préalablement dégazée à l'argon, sont ajoutés 7 g de 6-chloro 2,4-diaminopyrimidine 3-oxyde. Après dissolution, 4,58 g de thiométhylate de sodium sont ajoutés par petites fractions. Le mélange réactionnel est porté 5 heures au reflux puis est filtré sur verre fritté à température ambiante. Le précipité obtenu est lavé à l'eau et est séché sous vide.
Rendement = 80%.
Point de fusion = 276°C.

| Analyse élémentaire pour C₅H₈N₄OS ; PM = 172. | | | | | |
|---|---|---|---|---|---|
| | C | H | N | O | S |
| Calculé | 34,87 | 4,68 | 33,53 | 9,29 | 18,62 |
| Trouvé | 34,80 | 4,69 | 32,55 | 9,38 | 18,65 |

Les spectres de RMN ¹H et de masse sont en accord avec la structure attendue.

### EXEMPLE 9

### Préparation de 6-(beta amino éthylthio)2,4-diamino pyrimidine 3-oxyde.

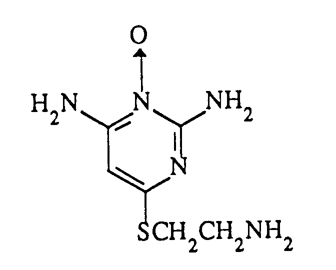

### Mode opératoire :

A une solution de 1 litre de méthanol absolu, contenant 31,2 g de chlorhydrate de cystéamine et préalablement dégazée à l'argon, sont ajoutés 29,7 g de méthylate de sodium de manière à maintenir la température inférieure ou égale à 30°C. Le mélange réactionnel est porté au reflux, puis 40 g de chloro 6-diamino 2,4-pyrimidine 3-oxyde sont ajoutés par petites fractions. Le mélange réactionnel est porté au reflux pendant 10 heures puis est filtré à chaud. Le filtrat est évaporé sous vide puis purifié sur colonne de silice dans le dichlorométhane en augmentant progressivement la polarité par du méthanol. Les fractions pures sont évaporées sous vide et le résidu est recristallisé dans le minimum d'éthanol bouillant. On obtient 15 g de poudre blanc-cassé.
Rendement : 30%.
Point de fusion = 212°C.

| Analyse élémentaire pour C₆H₁₁N₅ OS ; PM = 201. | | | |
|---|---|---|---|
| | C | H | N |
| Calculé | 35,81 | 5,51 | 34,80 |
| Trouvé | 35,37 | 5,34 | 34,04 |

Les spectres de RMN ¹H et de masse sont en accord avec la structure attendue.

### EXEMPLE 10

### Préparation de 6-(beta hydroxy éthylthio)2,4-diamino pyrimidine 3-oxyde.

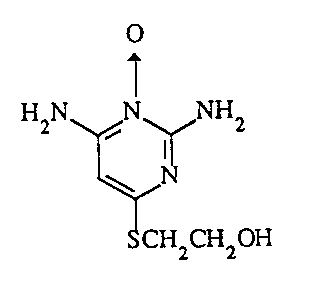

### Mode opératoire :

A une solution de 200 cm³ de méthanol absolu, contenant 8,8 cm³ de mercaptoéthanol et préalablement dégazée à l'argon, sont ajoutés 6,8 g de méthylate de sodium de manière a maintenir la température inférieure ou égale à 30°C. Le mélange réactionnel est porté au reflux puis 20 g de 6-chloro 2,4-diamino pyrimidine 3-oxyde sont ajoutés par petites fractions. Le mélange réactionnel est porté 2x8 heures au reflux puis est filtré sur verre fritté à température ambiante. Le précipité obtenu est lavé à l'eau, séché sous vide, puis recristallisé dans le minimum de diméthylsulfoxyde porté à température de 150°C. On obtient 12 g de poudre blanche.
Rendement = 50%.
Point de fusion = 245°C.

| Analyse élémentaire pour C₆H₁₀N₄O₂S 0,25 H₂O ; PM = 206,7. | | | | | |
|---|---|---|---|---|---|
| | C | H | N | O | S |
| Calculé | 34,86 | 5,12 | 27,10 | 17,41 | 15,51 |
| Trouvé | 34,40 | 5,34 | 26,71 | 16,90 | 15,76 |

Les spectres de RMN ¹H et de masse sont en accord avec la structure attendue.

### EXEMPLE 11

### Préparation du 2-amino 4-isobutylamino 5-chloro 6-éthoxy-pyrimidine 3-oxyde.

### Etape 1 : 2-amino 4-isobutylamino 6-chloropyrimidine.

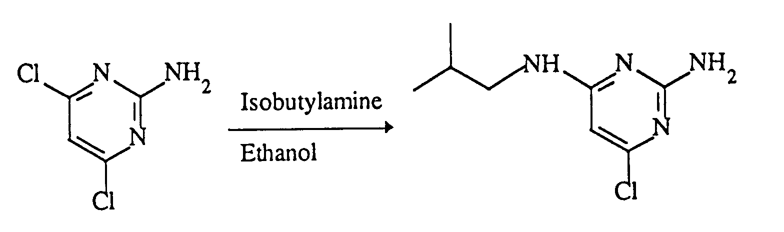

On place 50 g de 2-amino 4,6-dichloropyrimidine en suspension dans 250 ml d'éthanol. On additionne 45,65 g d'isobutylamine. Le milieu réactionnel est porté au reflux pendant 3 heures. On évapore à sec. Le résidu huileux est repris dans 250 ml d'eau. On agite vigoureusement 1 heure. Le précipité formé est filtré sur verre fritté, lavé par 100 ml d'eau, puis séché sous vide sur anhydride phosphorique. On recristallise dans 500 ml d'éther isopropylique. On obtient 50,15 g de 2-amino 4-isobutylamino 6-chloropyrimidine.
Rendement = 82%.

Le spectre de masse est en accord avec la structure attendue.

### Etape 2 : 2-amino 4-isobutylamino 6-chloro pyrimidine 3-oxyde.

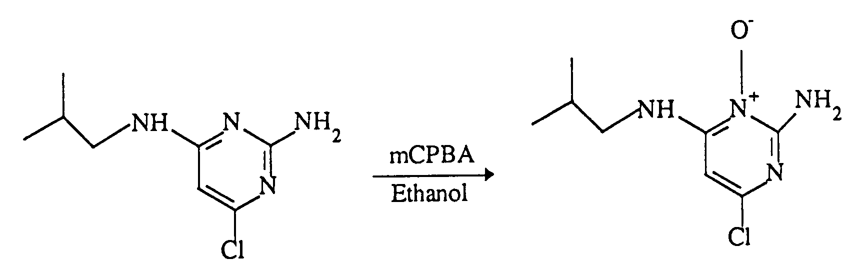

On dissout 34,70 g de 2-amino 4-isobutylamino 6-chloro pyrimidine dans 250 ml d'éthanol. On additionne, goutte à goutte et en maintenant la température du milieu réactionnel à 20°C, 81,50 g d'acide métachloroperbenzoïque on solution dans 350 ml d'éthanol. Après 2 h 30 d'agitation, le précipité formé est filtré sur verre fritté, lavé par 100 ml d'éther éthylique, puis repris dans 100 ml d'eau. On ajuste le pH à 8 par addition de soude concentrée. Après 1 heure d'agitation, le précipité est filtré sur verre fritté, lavé par 3 x 50 ml d'eau, séché sous vide sur anhydride phosphorique à 50°C. On obtient 11,30 g de 2-amino 4-isobutylamino 6-chloro pyrimidine 3-oxyde.
Rendement = 30%.

| Analyse élémentaire pour C₈H₁₃N₄ O Cl: M = 216,5. | | | | | |
|---|---|---|---|---|---|
| | C | H | N | O | Cl |
| Calculé | 44,34 | 6,00 | 25,87 | 7,39 | 16,40 |
| Trouvé | 44,52 | 5,96 | 25,75 | 7,46 | 16,41 |

Le spectre de masse est en accord avec la structure attendue.

### Etape 3 : 2-amino 4-isobutylamino 5,6-dichloro pyrimidine 3-oxyde.

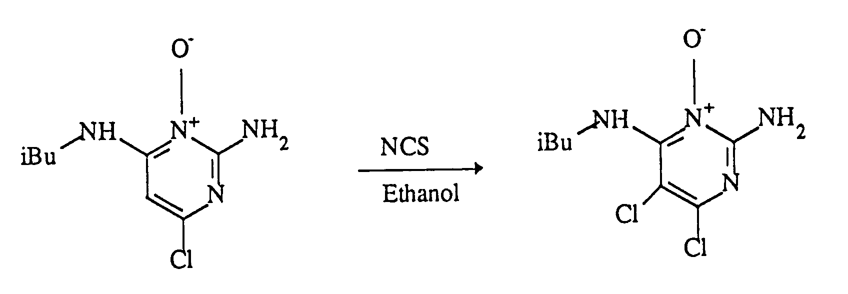

On place 6,30 g de 2-amino 4-isobutylamino 6-chloro pyrimidine 3-oxyde en suspension dans 110 ml d'éthanol. On additionne 4,85 g de N-chlorosuccinimide. Le milieu réactionnel est porté au reflux 2 heures puis évaporé à sec. Le résidu est repris dans 50 ml d'eau. On ajuste le pH à 8 par addition de soude concentrée. Le précipité formé est filtré sur verre fritté, lavé à l'eau jusqu'à neutralité, séché sous vide sur anhydride phosphorique. On le recristallise dans 150 ml d'éthanol. On obtient 4,30 g de 2-amino 4-isobutylamino 5,6-dichloro pyrimidine 3-oxyde.
Rendement = 59%.

| Analyse élémentaire pour C₈H₁₂N₄ O Cl₂ ; M = 251. | | | | | |
|---|---|---|---|---|---|
| | C | H | N | O | Cl |
| Calculé | 38,25 | 4,78 | 22,31 | 6,38 | 28,29 |
| Trouvé | 38,37 | 4,81 | 22,26 | 6,53 | 28,29 |

Le spectre de masse est en accord avec la structure attendue.

### Etape 4 : 2-amino 4-isobutylamino 5-chloro 6-éthoxy pyrimidine 3-oxyde.

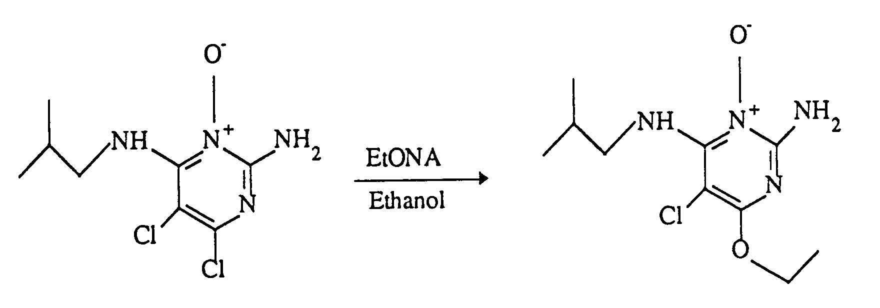

Sous bullage d'argon, on dissout 400 mg de sodium dans 30 ml d'éthanol. On additionne par petites spatules 3 g de 2-amino 4-isobutylamino 5,6-dichloro pyrimidine 3-oxyde. Le milieu réactionnel est porté au reflux pendant 2 heures puis évaporé à sec. Le résidu est repris dans 10 ml d'eau. Après 1 heure d'agitation, le précipité formé est filtré sur verre fritté, lavé par 3 x 10 ml d'eau, séché sous vide sur anhydride phosphorique. On recristallise dans 10 ml d'acétonitrile. On obtient 2,40 g de 2-amino 4-isobutylamino 5-chloro 6-éthoxy pyrimidine 3-oxyde.
Rendement = 77%.

| Analyse élémentaire pour C₁₀H₁₇N₄O₂Cl ; M = 260,5. | | | | | |
|---|---|---|---|---|---|
| | C | H | N | O | Cl |
| Calculé | 46,07 | 6,52 | 21,50 | 12,28 | 13,63 |
| Trouvé | 46,19 | 6,61 | 21,63 | 12,27 | 13,48 |

Les spectres de RMN ¹H et de masse sont en accord avec la structure attendue.

### EXEMPLE 12

### Préparation de 2-amino 4-isobutylamino 5-chloro 6-thiométhyl pyrimidine 3-oxyde.

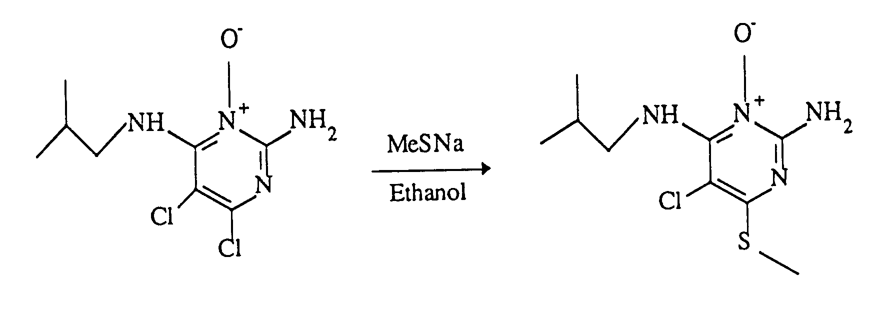

### Mode opératoire :

On place 2,5 g de 2-amino 4-isobutylamino 5,6-dichloro pyrimidine 3-oxyde préparé selon les 3 premières étapes du procédé de préparation de l'exemple 11, en suspension dans 25 ml d'éthanol. On additionne 800 mg de thiométhylate de sodium. Le milieu réactionnel est porté au reflux 5 heures puis évaporé à sec. Le résidu est repris dans 20 ml d'eau. On ajuste à pH 1 par addition d'acide chlorhydrique concentré. Après 1/2 heure d'agitation, le précipité formé est filtré sur verre fritté puis repris dans 10 ml d'eau. Le pH est ajusté à 8 par addition de soude concentrée. La solution est extraite par 2 x 20 ml d'acétate d'éthyle. La phase organique est séchée sur sulfate de sodium et évaporée à sec. Le résidu est recristallisé dans 17 ml d'un mélange éther isopropylique 9/acétone 1. On obtient 1 g de 2-amino 4-isobutylamino 5-chloro 6-thiométhyl pyrimidine 3-oxyde.
Rendement = 38%.

| Analyse élémentaire pour C₉H₁₅N₄O S Cl; M = 262,5. | | | | | | |
|---|---|---|---|---|---|---|
| | C | H | N | O | S | Cl |
| Calculé | 41,14 | 5,71 | 21,33 | 6,09 | 12,19 | 13,52 |
| Trouvé | 41,18 | 5,74 | 21,33 | 6,29 | 12,25 | 13,62 |

Les spectres de RMN ¹H et de masse sont en accord avec la structure attendue.

### EXEMPLES DE FORMULATION

### EXEMPLE 1

### LOTION CAPILLAIRE

| | |
|---|---|
| - 2,4-diamino 5-chloro 6-butyloxy pyrimidine 3-oxyde | 1,5 g |
| - Ethanol absolu | 92,3 g |
| - Propylèneglycol | 6,2 g |

### EXEMPLE 2

### LOTION

| | |
|---|---|
| - 2,4-diamino 6-thiophényl pyrimidine 3-oxyde | 0,40 g |
| - Ethanol absolu | 93,3 g |
| - Propylèneglycol | 6,3 g |

### EXEMPLE 3

### LOTION

| | |
|---|---|
| - 2,4-diamino 5-chloro 6-butyloxy pyrimidine 3-oxyde | 0,9 g |
| - Propylèneglycol | 22,6 g |
| - Ethanol | 43,9 g |
| -Eau | qsp 100,0 g |

### EXEMPLE 4

### LOTION

| | |
|---|---|
| - 2,4-diamino 6-thiophényl pyrimidine 3-oxyde | 0,9 g |
| - Propylèneglycol | 22,6 g |
| - Ethanol | 43,9 g |
| - Eau | qsp 100,0 g |

Ces compositions sont appliquées sur les zones alopéciques du cuir chevelu à raison de une fois par jour pendant 4 mois.

## Revendications (Revendications pour l'(les) Etat(s) contractant(s) suivant(s): AT, BE, CH, DE, DK, FR, GB, GR, IT, LI, NL, PT, SE)

1. Composition pour freiner la chute des cheveux et pour induire et stimuler leur croissance, caractérisée par le fait qu'elle contient dans un milieu physiologiquement acceptable, au moins un composé répondant à la formule suivante : 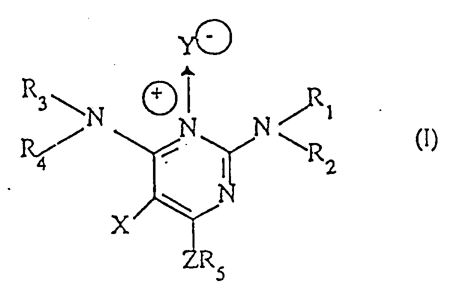 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone;
X désigne un atome d'hydrogène, un atome d'halogène,
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃;
ainsi que ses sels d'addition d'acides physiologiquement acceptables, sous réserve que le composé de formule (I) soit différent du 2,4-diamino 6 hydroxy 5 bromopyrimidine et du 2,4 diamino 6 thiophénylpyrimidine 3-oxi.

2. Composition selon la revendication 1, caractérisée par le fait que dans la formule (I), les radicaux alkyle en C₁-C₆ sont choisis parmi méthyle, éthyle, propyle, n-butyle, n-pentyle, n-hexyle; les radicaux alkyle en C₁-C₁₂ sont choisis parmi méthyle, éthyle, propyle, n-butyle, n-pentyle, n-hexyle, 2-éthylhexyle, octyle, décyle, dodécyle; les groupements alcényle en C₃-C₁₂ sont choisis parmi allyle, butényle, hexényle, décényle, dodécényle; les radicaux aryle ou aralkyle sont choisis parmi phényle, benzyle; les atomes d'halogène sont choisis parmi le chlore, le brome, le fluor ou l'iode.

3. Composition selon la revendication 1 ou 2, caractérisée par le fait que le composé de formule (I) est choisi parmi ceux pour lesquels Z désigne le soufre; X désigne le chlore, R₁, R₂, R₃, R₄ désignent hydrogène et R₅ désigne méthyle, éthyle, n-butyle, phényle, 2-hydroxyéthyle, 2-aminoéthyle.

4. Composition selon la revendication 1 ou 2, caractérisé par le fait que le composé de formule (I) est le 2,4-diamino 5-chloro 6-n-butyloxypyrimidine 3-oxyde.

5. Composition pharmaceutique ou cosmétique, destinée à être utilisée en application topique, caractérisée par le fait qu'elle contient dans un milieu physiologiquement acceptable, au moins un composé de formule (I) tel que défini dans l'une quelconque des revendications 1 à 4.

6. Composition selon la revendication 5, caractérisée par le fait qu'elle se présente sous forme d'onguent, de teinture, de crème, de pommade, de poudre, de timbre, de tampon imbibé, de solution, d'émulsion, de dispersion vésiculaire, de lotion, de gel, de spray ou de suspension anhydre ou aqueuse, en vue de son application pharmaceutique et qu'elle contient au moins un composé tel que défini dans l'une quelconque des revendications 1 à 3.

7. Composition pharmaceutique selon la revendication 6, caractérisée par le fait que le composé de formule (I) est présent dans des concentrations comprises entre 0,1 et 10% en poids par rapport au poids total de la composition.

8. Composition destinée à être utilisée en cosmétique, telle que définie dans la revendication 5, caractérisée par le fait qu'elle se présente sous forme de lotion, de gel, de savon, de shampooings d'aérosol ou de mousse et qu'elle contient, dans un support. cosmétiquement acceptable, au moins un composé tel que défini dans l'une quelconque des revendications 1 à 3, à une concentration comprise entre 0,01 et 5% en poids.

9. Composition selon l'une quelconque des revendications 5 à 8, caractérisée par le fait qu'elle contient en outre des agents hydratants et des agents antiséborrhéiques.

10. Composition selon l'une quelconque des revendications 5 à 9, caractérisée par le fait qu'elle contient également des agents améliorant encore l'activité des composés de formule (I) au niveau de la repousse et/ou du freinage de la chute des cheveux.

11. Composition selon la revendication 10, caractérisée par le fait qu'elle contient à titre d'agents améliorant encore l'activité de la repousse et/ou du freinage de la chute des cheveux, des esters d'acide nicotinique, des agents anti-inflammatoires stéroïdiens ou non-stéroïdiens, des rétinoïdes, des agents antibactériens, des agents antagonistes du calcium, des hormones, des agents anti-androgènes, des capteurs de radicaux OH.

12. Composition selon la revendication 10, caractérisée par le fait qu'elle contient à titre de composés améliorant encore l'activité sur la repousse et/ou le freinage de la chute des cheveux, des composés choisis parmi le Diazoxide, la Spiroxasone, les phospholipides, les acides linoléique et linolénique, l'acide salicylique et ses dérivés, les acides hydroxycarboxyliques ou cétocarboxyliques, leurs esters, les lactones et leurs sels correspondants, l'anthraline, les caroténoïdes, les acides eicosatétraynoïque-5,8,11,14, et eicosatriynoïque-5,8,11, leurs esters et amides.

13. Composition selon l'une quelconque des revendications 5 à 12, caractérisée par le fait qu'elle contient également des agents tensio-actifs choisis parmi les agents tensio-actifs non-ioniques et amphotères.

14. Composition selon l'une quelconque des revendications 5 à 13, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

15. Composition selon la revendication 14, caractérisée par le fait que les solvants sont choisis parmi les alcools inférieurs en C₁-C₄, les alkylèneglycols et les alkyléthers de mono- et de dialkylèneglycol.

16. Composition selon l'une quelconque des revendications 5 à 15, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants des filtres UV-A et UV-B, des antioxydants.

17. Composés de formule : 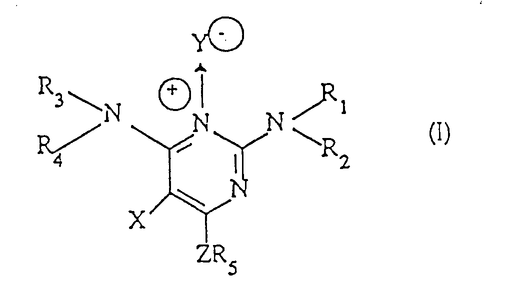 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone ;
X désigne un atome d'hydrogène, un atome d'halogène;
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃;
et les sels d'addition d'acides cosmétiquement et pharmaceutiquement acceptables; à l'exception des composés 2,4-diamino 6-hydroxy 5-bromopyrimidine 3-oxyde et 2,4-diamino 6-thiophénylpyrimidine 3-oxyde et leurs sels d'addition d'acides.

18. Composés tels que définis dans la revendication 17, pour utilisation comme substance thérapeutique.

19. Utilisation des composés de formule (I) tels que définis dans la revendication 1, pour la préparation d'un médicament destiné au traitement de l'alopécie, de la pelade, de la chute des cheveux, des dermatites desquamantes.

20. Procédé de traitement cosmétique des cheveux et du cuir chevelu, caractérisé par le fait que l'on applique une composition telle que définie dans les revendications 1 à 16.

21. Composition pour utilisation comme médicament pour freiner la chute des cheveux et pour induire et stimuler leur croissance, caractérisée par le fait qu'elle contient dans, un milieu physiologiquement acceptable, au moins un composé répondant à la formule suivante : 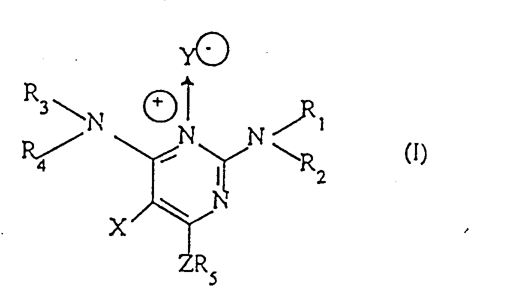 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone ;
X désigne un atome d'hydrogène, un atome d'halogène,
Z désigne le soufre ou l'oxygène ; sous réserve que Z désigne le soufre lorsque X désigne l'hydrogène ou lorsque R₅ désigne un radical aryle;
Y désigne l'oxygène ou OSO₃;
ainsi que ses sels d'addition d'acides physiologiquement acceptables.

## Revendications (Revendications pour l'(les) Etat(s) contractant(s) suivant(s): ES)

1. Composition pour freiner la chute des cheveux et pour induire et stimuler leur croissance, caractérisée par le fait qu'elle contient dans un milieu physiologiquement acceptable, au moins un composé répondant à la formule suivante : 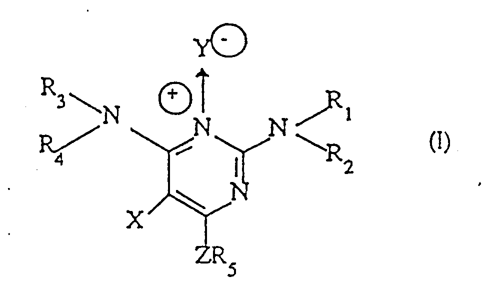 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone ;
X désigne un atome d'hydrogène, un atome d'halogène;
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃;
ainsi que ses sels d'addition d'acides physiologiquement acceptables, sous réserve que le composé de formule (I) soit différent du 2,4 diamino 6 hydroxy 5-bromopyrimidine et du 2,4-diamino 6 thiophénylpyrimidine 3-oxi.

2. Composition selon la revendication 1, caractérisée par le fait que dans la formule (I), les radicaux alkyle en C₁-C₆ sont choisis parmi méthyle, éthyle, propyle, n-butyle, n-pentyle, n-hexyle; les radicaux alkyle en C₁-C₁₂ sont choisis parmi méthyle, éthyle, propyle, n-butyle, n-pentyle, n-hexyle, 2-éthylhexyle, octyle, décyle, dodécyle; les groupements alcényle en C₃-C₁₂ sont choisis parmi allyle, butényle, hexényle, décényle, dodécényle; les radicaux aryle ou aralkyle sont choisis parmi phényle, benzyle; les atomes d'halogène sont choisis parmi le chlore, le brome, le fluor ou l'iode.

3. Composition selon la revendication 1 ou 2, caractérisée par le fait que le composé de formule (I) est choisi parmi ceux pour lesquels Z désigne le soufre; X désigne le chlore R₁, R₂, R₃, R₄ désignent hydrogène et R₅ désigne méthyle, éthyle, n-butyle, phényle, 2-hydroxyéthyle, 2-aminoéthyle.

4. Composition selon la revendication 1 ou 2, caractérisé par le fait que le composé de formule (I) est le 2,4-diamino 5-chloro 6-n-butyloxypyrimidine 3-oxyde.

5. Composition pharmaceutique ou cosmétique, destinée à être utilisée en application topique, caractérisée par le fait qu'elle contient dans un milieu physiologiquement acceptable, au moins un composé de formule (I) tel que défini dans l'une quelconque des revendications 1 à 4.

6. Composition selon la revendication 5, caractérisée par le fait qu'elle se présente sous forme d'onguent, de teinture, de crème, de pommade, de poudre, de timbre, de tampon imbibé, de solution, d'émulsion, de dispersion vésiculaire, de lotion, de gel, de spray ou de suspension anhydre ou aqueuse, en vue de son application pharmaceutique et qu'elle contient au moins un composé tel que défini dans l'une quelconque des revendications 1 à 3.

7. Composition destinée à être utilisée en cosmétique, telle que définie dans la revendication 5, caractérisée par le fait qu'elle se présente sous forme de lotion, de gel, de savon, de shampooing, d'aérosol ou de mousse et qu'elle contient, dans un support cosmétiquement acceptable, au moins un composé tel que défini dans l'une quelconque des revendications 1 à 3, à une concentration comprise entre 0,01 et 5% en poids.

8. Composition selon l'une quelconque des revendications 5 à 7, caractérisée par le fait qu'elle contient en outre des agents hydratants et des agents antiséborrhéiques.

9. Composition selon l'une quelconque des revendications 5 à 8, caractérisée par le fait qu'elle contient également des agents améliorant encore l'activité des composés de formule (I) au niveau de la repousse et/ou du freinage de la chute des cheveux.

10. Composition selon la revendication 9, caractérisée par le fait qu'elle contient à titre d'agents améliorant encore l'activité de la repousse et/ou du freinage de la chute des cheveux, des esters d'acide nicotinique, des agents anti-inflammatoires stéroïdiens ou non-stéroïdiens, des rétinoïdes, des agents antibactériens, des agents antagonistes du calcium, des hormones, des agents anti-androgènes, des capteurs de radicaux OH.

11. Composition selon la revendication 9, caractérisée par le fait qu'elle contient à titre de composés améliorant encore l'activité sur la repousse et/ou le freinage de la chute des cheveux, des composés choisis parmi le Diazoxide, la Spiroxasone, les phospholipides, les acides linoléique et linolénique, l'acide salicylique et ses dérivés, les acides hydroxycarboxyliques ou cétocarboxyliques, leurs esters, les lactones et leurs sels correspondants, l'anthraline, les caroténoïdes, les acides eicosatétraynoïque-5,8,11,14, et eicosatriynoïque-5,8,11, leurs esters et amides.

12. Composition selon l'une quelconque des revendications 5 à 11, caractérisée par le fait qu'elle contient également des agents tensio-actifs choisis parmi les agents tensio-actifs non-ioniques et amphotères.

13. Composition selon l'une quelconque des revendications 5 à 12, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

14. Composition selon la revendication 13, caractérisée par le fait que les solvants sont choisis parmi les alcools inférieurs en C₁-C₄, les alkylèneglycols et les alkyléthers de mono- et de dialkylèneglycol.

15. Composition selon l'une quelconque des revendications 5 à 14, caractérisée par le fait que le milieu physiologiquement acceptable est épaissi au moyen d'agents épaississants et/ou gélifiants et contient des agents conservateurs, des agents stabilisants, des agents, régulateurs de pH, des agents modificateurs de pression osmotique, des agents émulsifiants des filtres UV-A et UV-B, des antioxydants.

16. Procédé de préparation des composés de formule : 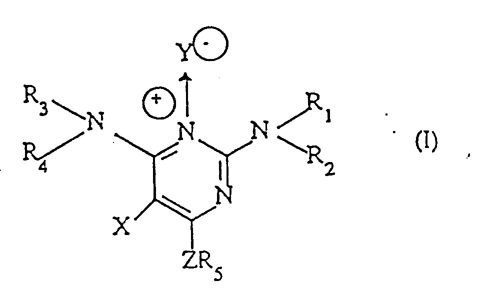 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone ;
X désigne un atome d'hydrogène, un atome d'halogène,;
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃; et les sels d'addition d'acides cosmétiquement et pharmaceutiquement acceptables; à l'exception des composés 2,4-diamino 6-hydroxy 5-bromopyrimidine 3-oxyde et 2,4-diamino 6-thiophénylpyrimidine 3-
oxyde et leurs sels d'addition d'acides, caractérisé par le fait que :
A) Pour obtenir les composés de formule (I) dans laquelle X désigne hydrogène et Y désigne oxygène, on fait réagir un composé de formule (II) : 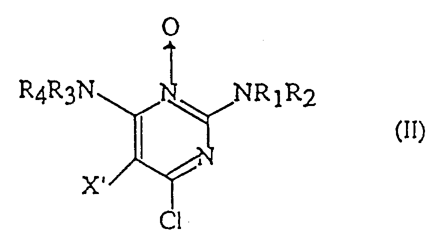 dans laquelle :
R₁ et R₃ désignent hydrogène;
R₂ et R₄ désignent un hydrogène ou un alkyle en C₁- C₄;
X' désigne hydrogène,
avec un alcoolate ^{⊖}OR₅, W^{⊕} dans l'alcool R₅OH où R₅ a la même signification indiquée ci-dessus et W désigne un métal alcalin ou avec un thiolate ^{⊖}SR₅, W^{⊕} en présence de diméthylformamide;
B) Pour obtenir les composés de formule (I) dans laquelle X désigne halogène et Y l'oxygène, on fait réagir les composés de formule (II) définie ci-dessus avec un agent halogénant en présence d'un alcool;
F) Pour obtenir les composés de formule (I) dans laquelle Y désigne OSO₃^{⊖} , on effectue sur les composés de formule (I) où Y désigne oxygène, une réaction de sulfatation avec un complexe de soufrepyridine, de trioxyde de soufre-triéthylamine ou de trioxyde de soufreéthyldiisopropylamine en présence d'un solvant pendant 1 à 24 heures entre 0 et 25°C.

17. Utilisation des composés de formule (I) tels que définis dans la revendication 1, pour la préparation d'un médicament destiné au traitement de l'alopécie, de la pelade, de la chute des cheveux, des dermatites desquamantes.

18. Procédé de traitement cosmétique des cheveux et du cuir chevelu, caractérisé par le fait que l'on applique une composition telle que définie dans les revendications 1 à 14.

19. Procédé de préparation d'une composition pour freiner la chute des cheveux et stimuler leur croissance, caractérisé par le fait que l'on incorpore dans un milieu physiologiquement acceptable, au moins un composé de formule : 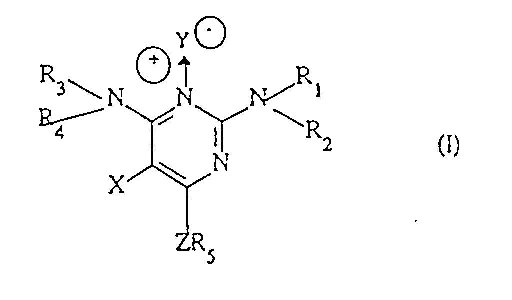 dans laquelle :
R₁ et R₃ désignent un atome d'hydrogène
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle on C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone ;
X désigne un atome d'hydrogène, un atome d'halogène;
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃;
ainsi que ses sels d'addition d'acides physiologiquement acceptables, sous réserve que le composé de formule (I) soit différent du 2,4 diamino 6 hydroxy 5-bromopyrimidine et du 2,4-diamino 6 thiophénylpyrimidine 3-oxi.

20. Composition pour utilisation comme médicament pour freiner la chute des cheveux et pour induire et stimuler leur croissance, caractérisée par le fait qu'elle contient dans un milieu physiologiquement acceptable, au moins un composé répondant à la formule suivante : 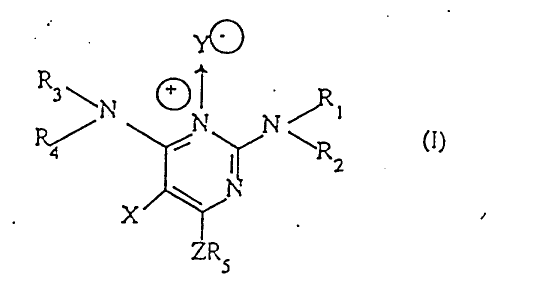 dans laquelle :
R₁ et R₃ désignent un atome d'hydrogène;
R₂ et R₄, identiques ou différents, représentent un atome d'hydrogène ou un alkyle en C₁-C₄ ;
R₅ désigne un atome d'hydrogène, un radical alkyle en C₁-C₁₂, un radical alcényle en C₃-C₁₂, un radical cycloalkyle en C₃-C₈, un radical aryle choisi de préférence parmi phényl et tolyle, un radical arylalkyle choisi de préférence parmi benzyle, un radical hydroxyalkyle ou aminoalkyle dans lequel le radical alkyle possède 1 à 6 atomes de carbone ;
X désigne un atome d'hydrogène, un atome d'halogène;
Z désigne le soufre ou l'oxygène ; sous réserve que Z désigne le soufre lorsque X désigne l'hydrogène ou lorsque R₅ désigne un radical aryle ;
Y désigne l'oxygène ou OSO₃;
ainsi que ses sels d'addition d'acides physiologiquement acceptables.

## Claims (Claims for the following Contracting State(s): AT, BE, CH, DE, DK, FR, GB, GR, IT, LI, NL, PT, SE)

1. Composition for slowing down hair loss and for inducing and stimulating its growth, characterized in that it comprises, in a physiologically acceptable medium, at least one compound corresponding to the following formula: 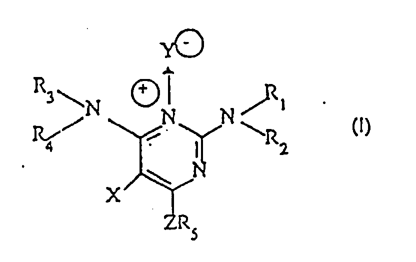 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen, with the proviso that Z denotes sulphur when X denotes hydrogen or when R₅ denotes an aryl radical;
Y denotes oxygen or OSO₃;
as well as its addition salts with physiologically acceptable acids, with the proviso that the compound of formula (I) is other than 2,4-diamino-6-hydroxy-5-bromopyrimidine and 2,4-diamino-6-thiophenylpyrimidine 3-oxi [sic].

2. Composition according to Claim 1, characterized in that, in the formula (I), the C₁-C₆ alkyl radicals are chosen from methyl, ethyl, propyl, n-butyl, n-pentyl and n-hexyl; the C₁-C₁₂ alkyl radicals are chosen from methyl, ethyl, propyl, n-butyl, n-pentyl, n-hexyl, 2-ethylhexyl, octyl, decyl and dodecyl; the C₃-C₁₂ alkenyl groups are chosen from allyl, butenyl, hexenyl, decenyl and dodecenyl; the aryl or aralkyl radicals are chosen from phenyl or benzyl; the halogen atoms are chosen from chlorine, bromine, fluorine or iodine.

3. Composition according to Claim 1 or 2, characterized in that the compound of formula (I) is chosen from those for which Z denotes sulphur; X denotes chlorine, R₁, R₂, R₃ and R₄ denote hydrogen and R₅ denotes methyl, ethyl, n-butyl, phenyl, 2-hydroxyethyl or 2-aminoethyl.

4. Composition according to Claim 1 or 2, characterized in that the compound of formula (I) is 2,4-diamino-5-chloro-6-n-butyloxypyrimidine 3-oxide.

5. Pharmaceutical or cosmetic composition, intended to be used in topical application, characterized in that it comprises, in a physiologically acceptable medium, at least one compound of formula (I) such as defined in any one of Claims 1 to 4.

6. Composition according to Claim 5, characterized in that it is present in the form of an anhydrous or aqueous, ointment, tincture, cream, pomade, powder, patch, impregnated pad, solution, emulsion, vesicular dispersion, lotion, gel, spray or suspension for the purpose of its pharmaceutical application and that it comprises at least one compound such as defined in any one of Claims 1 to 3.

7. Pharmaceutical composition according to Claim 6, characterized in that the compound of formula (I) is present in concentrations of between 0.1 and 10 % by weight in relation to the total weight of the composition.

8. Composition intended to be used in cosmetics, such as defined in Claim 5, characterized in that it is present in the form of a lotion, gel, soap, shampoo, aerosol or foam and that it comprises, in a cosmetically acceptable carrier, at least one compound such as defined in any one of Claims 1 to 3, at a concentration of between 0.01 and 5 % by weight.

9. Composition according to any one of Claims 5 to 8, characterized in that it additionally contains hydrating agents and antiseborrheic agents.

10. Composition according to any one of Claims 5 to 9, characterized in that it also contains agents for further improving the activity of the compounds of formula (I) as regards the regrowth and/or slowing down of hair loss.

11. Composition according to Claim 10, characterized in that it comprises, as agents for further improving the activity of regrowth and/or of slowing down of hair loss, nicotinic acid esters, steroidal or nonsteroidal antiinflammatory agents, retinoids, antibacterial agents, calcium antagonistic agents, hormones, antiandrogenic agents and OH radical scavengers.

12. Composition according to Claim 10, characterized in that it comprises, as compounds for further improving activity towards the regrowth and/or slowing down of hair loss, compounds chosen from diazoxide, spiroxasone, the phospholipids, linoleic and linolenic acids, salicylic acid and its derivatives, the hydroxycarboxylic or ketocarboxylic acids, their esters, the lactones and their corresponding salts, anthralin, the carotenoids, 5,8,11,14-eicosatetraynoic and 5,8,11-eicosatriynoic acids, their esters and amides.

13. Composition according to any one of Claims 5 to 12, characterized in that it also comprises surface-active agents chosen from the nonionic and amphoteric surface-active agents.

14. Composition according to any one of Claims 5 to 13, characterized in that the physiologically acceptable medium consists of water, a mixture of water and of one or more organic solvent(s) or of a mixture of organic solvents, the organic solvents being pharmaceutically or cosmetically acceptable.

15. Composition according to Claim 14, characterized in that the solvents are chosen from the C₁-C₄ lower alcohols, the alkylene glycols and the mono- and dialkylene glycol alkyl ethers.

16. Composition according to any one of Claims 5 to 15, characterized in that the physiologically acceptable medium is thickened by means of thickening and/or gelling agents and contains preserving agents, stabilizing agents, pH regulating agents, agents for modifiyng osmotic pressure, emulsifying agents, UV-A and UV-B filters or antioxidant agents.

17. Compounds of formula: 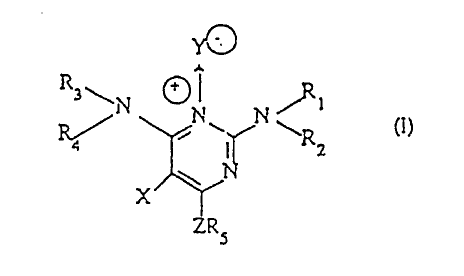 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen; with the proviso that Z denotes sulphur when X denotes hydrogen or when R₅ denotes an aryl radical;
Y denotes oxygen or OSO₃;
and the addition salts with cosmetically and pharmaceutically acceptable acids; with the exception of the compounds 2,4-diamino-6-hydroxy-5-bromopyrimidine 3-oxide and 2,4-diamino-6-thiophenylpyrimidine 3-oxide and their addition salts with acids.

18. Compounds such as defined in Claim 17, for use as therapeutic substance.

19. Use of the compounds of formula (I) such as defined in Claim 1, for the preparation of a medicament intended for the treatment of alopecia, pelade, hair loss or desquamative dermatitis.

20. Process for cosmetic treatment of the hair and the scalp, characterized in that a composition such as defined in Claims 1 to 16 is applied.

21. Composition for use as medicament for slowing down hair loss and for inducing and stimulating its growth, characterized in that it comprises, in a physiologically acceptable medium, at least one compound corresponding to the following formula: 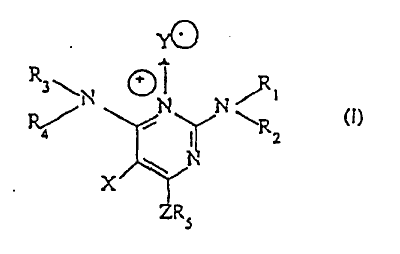 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen, with the proviso that Z denotes sulphur when X denotes hydrogen or when R₅ denotes an aryl radical;
Y denotes oxygen or OSO₃;
as well as its addition salts with physiologically acceptable acids.

## Claims (Claims for the following Contracting State(s): ES)

1. Composition for slowing down hair loss and for inducing and stimulating its growth, characterized in that it comprises, in a physiologically acceptable medium, at least one compound corresponding to the following formula: 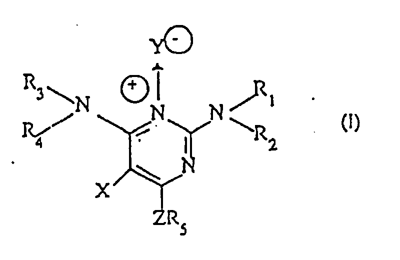 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen, with the proviso that Z denotes sulphur when X denotes hydrogen or when R₅ denotes an aryl radical;
Y denotes oxygen or OSO₃;
as well as its addition salts with physiologically acceptable acids, with the proviso that the compound of formula (I) is other than 2,4-diamino-6-hydroxy-5-bromopyrimidine and 2,4-diamino-6-thiophenylpyrimidine 3-oxi [sic].

2. Composition according to Claim 1, characterized in that, in the formula (I), the C₁-C₆ alkyl radicals are chosen from methyl, ethyl, propyl, n-butyl, n-pentyl and n-hexyl; the C₁-C₁₂ alkyl radicals are chosen from methyl, ethyl, propyl, n-butyl, n-pentyl, n-hexyl, 2-ethylhexyl, octyl, decyl and dodecyl; the C₃-C₁₂ alkenyl groups are chosen from allyl, butenyl, hexenyl, decenyl and dodecenyl; the aryl or aralkyl radicals are chosen from phenyl or benzyl; the halogen atoms are chosen from chlorine, bromine, fluorine or iodine.

3. Composition according to Claim 1 or 2, characterized in that the compound of formula (I) is chosen from those for which Z denotes sulphur; X denotes chlorine, R₁, R₂, R₃ and R₄ denote hydrogen and R₅ denotes methyl, ethyl, n-butyl, phenyl, 2-hydroxyethyl or 2-aminoethyl.

4. Composition according to Claim 1 or 2, characterized in that the compound of formula (I) is 2,4-diamino-5-chloro-6-n-butyloxypyrimidine 3-oxide.

5. Pharmaceutical or cosmetic composition, intended to be used in topical application, characterized in that it comprises, in a physiologically acceptable medium, at least one compound of formula (I) such as defined in any one of Claims 1 to 4.

6. Composition according to Claim 5, characterized in that it is present in the form of an anhydrous or aqueous, ointment, tincture, cream, pomade, powder, patch, impregnated pad, solution, emulsion, vesicular dispersion, lotion, gel, spray or suspension for the purpose of its pharmaceutical application and that it comprises at least one compound such as defined in any one of Claims 1 to 3.

7. Composition intended to be used in cosmetics, such as defined in Claim 5, characterized in that it is present in the form of a lotion, gel, soap, shampoo, aerosol or foam and that it comprises, in a cosmetically acceptable carrier, at least one compound such as defined in any one of Claims 1 to 3, at a concentration of between 0.01 and 5 % by weight.

8. Composition according to any one of Claims 5 to 7, characterized in that it additionally contains hydrating agents and antiseborrheic agents.

9. Composition according to any one of Claims 5 to 8, characterized in that it also contains agents for further improving the activity of the compounds of formula (I) as regards the regrowth and/or slowing down of hair loss.

10. Composition according to Claim 9, characterized in that it comprises, as agents for further improving the activity of regrowth and/or of slowing down of hair loss, nicotinic acid esters, steroidal or nonsteroidal antiinflammatory agents, retinoids, antibacterial agents, calcium antagonistic agents, hormones, antiandrogenic agents and OH radical scavengers.

11. Composition according to Claim 9, characterized in that it comprises, as compounds for further improving activity towards the regrowth and/or slowing down of hair loss, compounds chosen from diazoxide, spiroxasone, the phospholipids, linoleic and linolenic acids, salicylic acid and its derivatives, the hydroxycarboxylic or ketocarboxylic acids, their esters, the lactones and their corresponding salts, anthralin, the carotenoids, 5,8,11,14-eicosatetraynoic and 5,8,11-eicosatriynoic acids, their esters and amides.

12. Composition according to any one of Claims 5 to 11, characterized in that it also comprises surface-active agents chosen from the nonionic and amphoteric surface-active agents.

13. Composition according to any one of Claims 5 to 12, characterized in that the physiologically acceptable medium consists of water, a mixture of water and of one or more organic solvent(s) or of a mixture of organic solvents, the organic solvents being pharmaceutically or cosmetically acceptable.

14. Composition according to Claim 13, characterized in that the solvents are chosen from the C₁-C₄ lower alcohols, the alkylene glycols and the mono- and dialkylene glycol alkyl ethers.

15. Composition according to any one of Claims 5 to 14, characterized in that the physiologically acceptable medium is thickened by means of thickening and/or gelding agents and contains preserving agents, stabilizing agents, pH regulating agents, agents for modifiyng osmotic pressure, emulsifying agents, UV-A and UV-B filters or antioxidant agents.

16. Process for the preparation of compounds of formula: 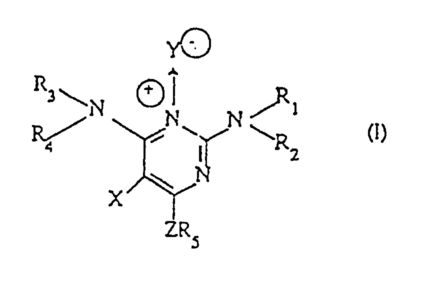 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen; with the proviso that Z denotes sulphur when X denotes hydrogen or when R₅ denotes an aryl radical;
Y denotes oxygen or OSO₃;
and the addition salts with cosmetically and pharmaceutically acceptable acids; with the exception of the compounds 2,4-diamino-6-hydroxy-5-bromopyrimidine 3-oxide and 2,4-diamino-6-thiophenylpyrimidine 3-oxide and their addition salts with acids, characterized in that:
A) In order to obtain the compounds of formula (I) in which X denotes hydrogen and Y denotes oxygen, a compound of formula (II): 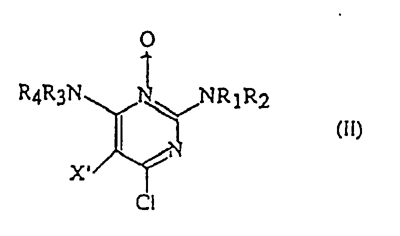 in which:
R₁ and R₃ denote hydrogen;
R₂ and R₄ denote a hydrogen or a C₁-C₄ alkyl;
X' denotes hydrogen;
is reacted with an alkoxide W^{+ -}OR₅ in the alcohol R₅OH, where R₅ has the same meaning indicated above and W denotes an alkali metal, or with a thiolate W^{+ -}SR₅ in the presence of dimethylformamide;
B) In order to obtain the compounds of formula (I) in which X denotes halogen and Y oxygen, the compounds of formula (II) which is defined above are reacted with a halogenating agent in the presence of an alcohol;
F) In order to obtain the compounds of formula (I) in which Y denotes OSO₃⁻, a sulphation reaction is carried out, on the compounds of formula (I) where Y denotes oxygen, with a sulphur-pyridine [sic], sulphur trioxide-triethylamine or sulphur trioxide-ethyldiisopropylamine complex in the presence of a solvent for 1 to 24 hours between 0 and 25°C.

17. Use of the compounds of formula (I) such as defined in Claim 1, for the preparation of a medicament intended for the treatment of alopecia, pelade, hair loss or desquamative dermatitis.

18. Process for cosmetic treatment of the hair and the scalp, characterized in that a composition such as defined in Claims 1 to 14 is applied.

19. Process for the preparation of a composition for slowing down hair loss and stimulating its growth, characterized in that at least one compound of formula: 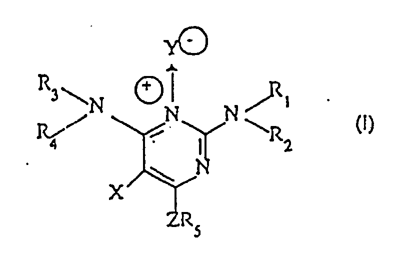 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen, with the proviso that Z denotes sulphur when X denotes hydrogen or when R₅ denotes an aryl radical;
Y denotes oxygen or OSO₃;
as well as its addition salts with physiologically acceptable acids, with the proviso that the compound of formula (I) is other than 2,4-diamino-6-hydroxy-5-bromopyrimidine and 2,4-diamino-6-thiophenylpyrimidine 3-oxi [sic], is incorporated in a physiologically acceptable medium.

20. Composition for use as medicament for slowing down hair loss and for inducing and stimulating its growth, characterized in that it comprises, in a physiologically acceptable medium, at least one compound corresponding to the following formula: 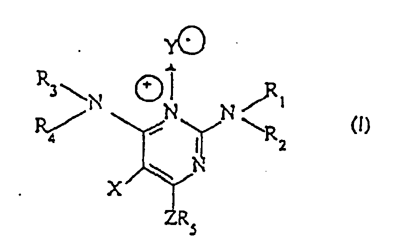 in which:
R₁ and R₃ denote a hydrogen atom;
R₂ and R₄, which are identical or different, represent a hydrogen atom or a C₁-C₄ alkyl group;
R₅ denotes a hydrogen atom, a C₁-C₁₂ alkyl radical, a C₃-C₁₂ alkenyl radical, a C₃-C₈ cycloalkyl radical, an aryl radical, preferably chosen from phenyl and tolyl, an arylalkyl radical, preferably chosen from benzyl, or a hydroxyalkyl or aminoalkyl radical in which the alkyl radical has 1 to 6 carbon atoms;
X denotes a hydrogen atom or a halogen atom;
Z denotes sulphur or oxygen, with the proviso that Z denotes sulphur when X denotes hydrogen or when R₅ denotes an aryl radical;
Y denotes oxygen or OSO₃;
as well as its addition salts with physiologically acceptable acids.

## Patentansprüche (Patentansprüche für folgende(n) Vertragsstaat(en): AT, BE, CH, DE, DK, FR, GB, GR, IT, LI, NL, PT, SE)

1. Zusammensetzung zur Verlangsamung von Haarausfall und zur Induzierung und Stimulierung des Haarwachstums,
dadurch gekennzeichnet, daß
sie in einem physiologisch akzeptablen Medium mindestens eine Verbindung der folgenden Formel enthält: 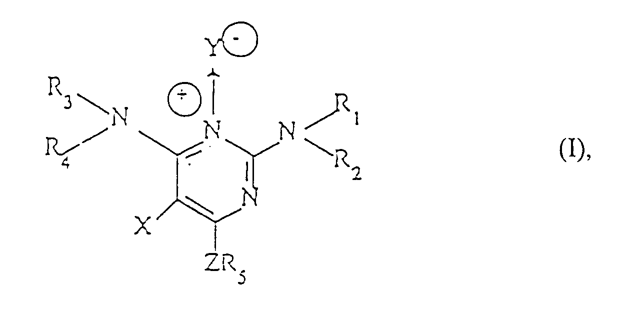 worin bedeuten:
R₁ und R₃ Wasserstoff;
R₂ und R₄, die identisch oder voneinander verschieden sind, Wasserstoff oder C₁₋₄-Alkyl,
R₅ Wasserstoff, C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist;
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie die Additionssalze dieser Verbindungen mit physiologisch akzeptablen Säuren, mit der Maßgabe, daß die Verbindung der Formel (I) von 2,4-Diamino-6-hydroxy-5-brompyrimidin-3-oxid und 2,4-Diamino-6-thiophenylpyrimidin-3-oxid verschieden ist.

2. Zusammensetzung nach Anspruch 1, dadurch gekennzeichnet, daß in der Formel (1) die C₁₋₆-Alkylgruppen unter Methyl, Ethyl, Propyl, n-Butyl, n-Pentyl und n-Hexyl, die C₁₋₁₂-Alkylgruppen unter Methyl, Ethyl, Propyl, n-Butyl, n-Pentyl, n-Hexyl, 2-Ethylhexyl, Octyl, Decyl und Dodecyl; die C₃₋₁₂-Alkenylgruppen unter Allyl, Butenyl, Hexenyl, Decenyl und Dodecenyl; die Aryl- oder Aralkylgruppen unter Phenyl und Benzyl; und die Halogenatome unter Chlor, Brom, Fluor oder Iod ausgewählt sind.

3. Zusammensetzung nach Anspruch 1 oder 2, dadurch gekennzeichnet, daß die Verbindung der Formel (I) unter den Verbindungen ausgewählt ist, worin Z Schwefel, X Chlor, die Gruppen R₁, R₂, R₃ und R₄ Wasserstoff und die Gruppe R₅ Methyl, Ethyl, n-Butyl, Phenyl, 2-Hydroxyethyl oder 2-Aminoethyl bedeuten.

4. Zusammensetzung nach Anspruch 1 oder 2, dadurch gekennzeichnet, daß die Verbindung der Formel (I) das 2,4-Diamino-5-chlor-6-n-butyloxypyrimidin-3-oxid ist.

5. Pharmazeutische oder kosmetische Zusammensetzung zur topischen Anwendung, dadurch gekennzeichnet, daß sie in einem physiologisch akzeptablen Medium mindestens eine Verbindung der Formel (I) nach einem der Ansprüche 1 bis 4 enthält.

6. Zusammensetzung nach Anspruch 5, dadurch gekennzeichnet, daß sie in Abhängigkeit von der pharmazeutischen Verwendung wasserfrei oder wäßrig als Salbe, Tinktur, Creme, Pomade, Pulver, Pflaster, durchtränkter Gazestreifen, Lösung, Emulsion, Vesikeldispersion, Lotion, Gel, Spray oder Suspension vorliegt und mindestens eine Verbindung nach einem der Ansprüche 1 bis 3 enthält.

7. Pharmazeutische Zusammensetzung nach Anspruch 6, dadurch gekennzeichnet, daß die Verbindung der Formel (I) in Konzentrationen von 0,1 bis 10 Gew.-%. bezogen auf das Gesamtgewicht der Zusammensetzung, vorliegt.

8. Zusammensetzung zur Verwendung in der Kosmetik nach Anspruch 5, dadurch gekennzeichnet, daß sie als Lotion, Gel, Seife, Haarwaschmittel, Aerosol oder Schaum vorliegt und in einem kosmetisch akzeptablen Träger mindestens eine Verbindung nach einem der Ansprüche 1 bis 3 in einer Konzentration von 0,01 bis 5 Gew.-% enthält.

9. Zusammensetzung nach einem der Ansprüche 5 bis 8, dadurch gekennzeichnet, daß sie feiner Hydratisierungsmittel und Antiseborrhöika enthält.

10. Zusammensetzung nach einem der Ansprüche 5 bis 9, dadurch gekennzeichnet, daß sie auch Mittel enthält, die die Wirksamkeit der Verbindungen der Formel (I) bezüglich des Haarwuchses und/oder der Verlangsamung des Haarausfalles verbessern.

11. Zusammensetzung nach Anspruch 10, dadurch gekennzeichnet, daß sie als Mittel zur Verbesserung der Wirksamkeit bezüglich des Haarwuchses und/oder der Verlangsamung des Haarausfalles Nicotinsäureester, steroidale oder nichtsteroidale entzündungshemmende Mittel, Retinoide, antibakterielle Mittel, Calcium-Antagonisten, Hormone, antiandrogene Mittel und Radikalfänger für OH-Radikale enthält.

12. Zusammensetzung nach Anspruch 10, dadurch gekennzeichnet, daß sie als Mittel, die die Wirksamkeit bezüglich des Haarwuchses und/oder der Verlangsamung des Haarausfalls verbessern, Verbindungen enthält, die unter Diazoxid, Spiroxason, Phospholipiden, Linolsäure und Linolensäure, Salicylsäure und ihren Derivaten, Hydroxycarbonsäuren oder Ketocarbonsäuren und ihren korrespondierenden Estern, Lactonen und Salzen, Anthralin, Carotinoiden, 5,8,11,14-Eicosatetraensäure und 5,8,11-Eicosatriensäure und deren Estern und Amiden ausgewählt ist.

13. Zusammensetzung nach einem der Ansprüche 5 bis 12, dadurch gekennzeichnet, daß sie auch grenzflächenaktive Stoffe enthält, die unter den nichtionischen und amphoteren grenzflächenaktiven Stoffen ausgewählt sind.

14. Zusammensetzung nach einem der Ansprüche 5 bis 13, dadurch gekennzeichnet, daß das physiologisch akzeptable Medium aus Wasser, einem Gemisch von Wasser und einem oder mehreren organischen Lösungsmitteln oder einem Gemisch von organischen Lösungsmitteln besteht, wobei die organischen Lösungsmittel pharmazeutisch oder kosmetisch akzeptabel sind.

15. Zusammensetzung nach Anspruch 14, dadurch gekennzeichnet, daß die Lösungsmittel unter den niederen C₁₋₄-Alkoholen, den Alkylenglykolen und Mono- und Dialkylenglykolalkylethern ausgewählt sind.

16. Zusammensetzung nach einem der Ansprüche 5 bis 15, dadurch gekennzeichnet, daß das physiologisch akzeptable Medium mit Verdickungsmitteln und/oder Gelbildnern verdickt ist und Konservierungsmittel, Stabilisatoren, pH-Regulatoren, Modifikatoren des osmotischen Drucks, Emulgatoren, UV-A- und UV-B-Filter und Antioxidantien enthält.

17. Verbindungen der Formel: 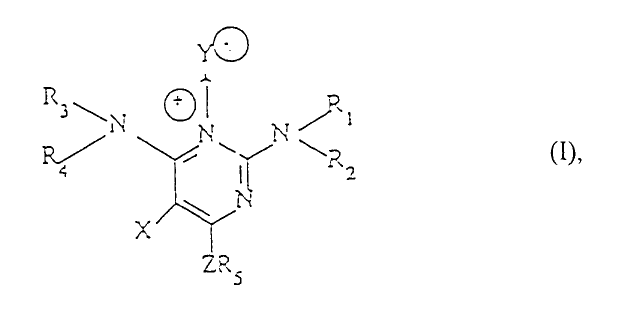 worin bedeuten:
R₁ und R₃ Wasserstoff;
R₂ und R₄, die identisch oder voneinander verschieden sind, Wasserstoff oder C₁₋₄-Alkyl,
R₅ Wasserstoff, C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist:
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie die Additionssalze dieser Verbindungen mit kosmetisch und pharmazeutisch akzeptablen Säuren, mit der Maßgabe, daß 2,4-Diamino-6-hydroxy-5-brompyrimidin-3-oxid und 2,4-Diamino-6-thiophenylpyrimidin-3-oxid und deren Additionssalze mit einer Säure ausgenommen sind.

18. Verbindungen nach Anspruch 17 zur Verwendung als therapeutisches Mittel.

19. Verwendung der Verbindungen der Formel (I) nach Anspruch 1 zur Herstellung eines Arzneimittels, das zur Behandlung von Alopezie, Pelade, Haarausfall und desquamativer Dermatitis verwendet werden soll.

20. Verfahren zur kosmetischen Behandlung der Haare und der Kopfhaut, dadurch gekennzeichnet, daß eine Zusammensetzung nach einem der Ansprüche 1 bis 16 aufgetragen wird.

21. Zusammensetzung zur Verwendung als Arzneimittel, um den Haarausfall zu verlangsamen und das Haarwachstum zu induzieren und zu stimulieren, dadurch gekennzeichnet, daß sie in einem physiologisch akzeptablen Medium mindestens eine Verbindung der folgenden Formel enthält: 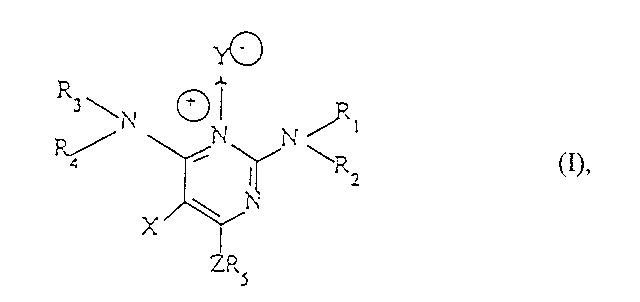 worin bedeuten:
R₁ und R₃ Wasserstoff;
R₂ und R₄, die identisch oder voneinander verschieden sind, Wasserstoff oder C₁₋₄-Alkyl,
R₅ Wasserstoff, C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist;
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie die Additionssalze dieser Verbindungen mit physiologisch akzeptablen Säuren.

## Patentansprüche (Patentansprüche für folgende(n) Vertragsstaat(en): ES)

1. Zusammensetzung zur Verlangsamung von Haarausfall und zur Induzierung und Stimulierung des Haarwachstums,
dadurch gekennzeichnet, daß
sie in einem physiologisch akzeptablen Medium mindestens eine Verbindung der folgenden Formel enthält: 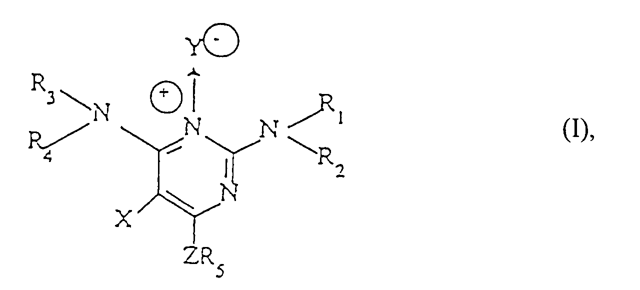 worin bedeuten:
R₁ und R₃ Wasserstoff;
R₂ und R₄, die identisch oder voneinander verschieden sind, Wasserstoff oder C₁₋₄-Alkyl,
R₅ Wasserstoff, C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist;
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie die Additionssalze dieser Verbindungen mit physiologisch akzeptablen Säuren, mit der Maßgabe, daß die Verbindung der Formel (I) von 2,4-Diamino-6-hydroxy-5-brompyrimidin-3-oxid und 2,4-Diamino-6-thiophenylpyrimidin-3-oxid verschieden ist.

2. Zusammensetzung nach Anspruch 1, dadurch gekennzeichnet, daß in der Formel (1) die C₁₋₆-Alkylgruppen unter Methyl, Ethyl, Propyl, n-Butyl, n-Pentyl und n-Hexyl, die C₁₋₁₂-Alkylgruppen unter Methyl, Ethyl, Propyl, n-Butyl, n-Pentyl, n-Hexyl, 2-Ethylhexyl, Octyl, Decyl und Dodecyl; die C₃₋₁₂-Alkenylgruppen unter Allyl, Butenyl, Hexenyl, Decenyl und Dodecenyl; die Aryl- oder Aralkylgruppen unter Phenyl und Benzyl; und die Halogenatome unter Chlor, Brom, Fluor oder Iod ausgewählt sind.

3. Zusammensetzung nach Anspruch 1 oder 2, dadurch gekennzeichnet, daß die Verbindung der Formel (I) unter den Verbindungen ausgewählt ist, worin Z Schwefel, X Chlor, die Gruppen R₁, R₂, R₃ und R₄ Wasserstoff und die Gruppe R₅ Methyl, Ethyl, n-Butyl, Phenyl, 2-Hydroxyethyl oder 2-Aminoethyl bedeutet.

4. Zusammensetzung nach Anspruch 1 oder 2, dadurch gekennzeichnet, daß die Verbindung der Formel (I) das 2,4-Diamino-5-chlor-6-n-butyloxypyrimidin-3-oxid ist.

5. Pharmazeutische oder kosmetische Zusammensetzung zur topischen Anwendung, dadurch gekennzeichnet, daß sie in einem physiologisch akzeptablen Medium mindestens eine Verbindung der Formel (I) nach einem der Ansprüche 1 bis 4 enthält.

6. Zusammensetzung nach Anspruch 5, dadurch gekennzeichnet, daß sie in Abhängigkeit von der pharmazeutischen Verwendung wasserfrei oder wäßrig als Salbe, Tinktur, Creme, Pomade, Pulver; Pflaster, durchtränkter Gazestreifen, Lösung, Emulsion, Vesikeldispersion, Lotion, Gel, Spray oder Suspension vorliegt und mindestens eine Verbindung nach einem der Ansprüche 1 bis 3 enthält.

7. Zusammensetzung zur Verwendung in der Kosmetik nach Anspruch 5, dadurch gekennzeichnet, daß sie als Lotion, Gel, Seife, Haarwaschmittel, Aerosol oder Schaum vorliegt und in einem kosmetisch akzeptablen Träger mindestens eine Verbindung nach einem der Ansprüche 1 bis 3 in einer Konzentration von 0,01 bis 5 Gew.-% enthält.

8. Zusammensetzung nach einem der Ansprüche 5 bis 7, dadurch gekennzeichnet, daß sie ferner Hydratisierungsmittel und Antiseborrhöika enthält.

9. Zusammensetzung nach einem der Ansprüche 5 bis 8, dadurch gekennzeichnet, daß sie auch Mittel enthält, die die Wirksamkeit der Verbindungen der Formel (I) bezüglich des Haarwuchses und/oder der Verlangsamung des Haarausfalles verbessern.

10. Zusammensetzung nach Anspruch 9, dadurch gekennzeichnet, daß sie als Mittel zur Verbesserung der Wirksamkeit bezüglich des Haarwuchses und/oder der Verlangsamung des Haarausfalles Nicotinsäureester, steroidale oder nichtsteroidale entzündungshemmende Mittel, Retinoide, antibakterielle Mittel, Calcium-Antagonisten, Hormone, antiandrogene Mittel und Radikalfänger für OH-Radikale enthält.

11. Zusammensetzung nach Anspruch 9, dadurch gekennzeichnet, daß sie als Mittel, die die Wirksamkeit bezüglich des Haarwuchses und/oder der Verlangsamung des Haarausfalls verbessern, Verbindungen enthält, die unter Diazoxid, Spiroxason, Phospholipiden, Linolsäure und Linolensäure, Salicylsäure und ihren Derivaten, Hydroxycarbonsäuren oder Ketocarbonsäuren und ihren korrespondierenden Estern, Lactonen und Salzen, Anthralin, Carotinoiden, 5,8,11,14-Eicosatetraensäure und 5,8,11-Eicosatriensäure und deren Estern und Amiden ausgewählt ist.

12. Zusammensetzung nach einem der Ansprüche 5 bis 11, dadurch gekennzeichnet, daß sie auch grenzflächenaktive Stoffe enthält, die unter den nichtionischen und amphoteren grenzflächenaktiven Stoffen ausgewählt sind.

13. Zusammensetzung nach einem der Ansprüche 5 bis 12, dadurch gekennzeichnet, daß das physiologisch akzeptable Medium aus Wasser, einem Gemisch von Wasser und einem oder mehreren organischen Lösungsmitteln oder einem Gemisch von organischen Lösungsmitteln besteht, wobei die organischen Lösungsmittel pharmazeutisch oder kosmetisch akzeptabel sind.

14. Zusammensetzung nach Anspruch 13, dadurch gekennzeichnet, daß die Lösungsmittel unter den niederen C₁₋₄-Alkoholen, den Alkylenglykolen und Mono- und Dialkylenglykolalkylethern ausgewählt sind.

15. Zusammensetzung nach einem der Ansprüche 5 bis 14, dadurch gekennzeichnet, daß das physiologisch akzeptable Medium mit Verdickungsmitteln und/oder Gelbildnern verdickt ist und Konservierungsmittel, Stabilisatoren, pH-Regulatoren, Modifikatoren des osmotischen Drucks, Emulgatoren, UV-A- und UV-B-Filter und Antioxidantien enthält.

16. Verfahren zur Herstellung von Verbindungen der Formel: 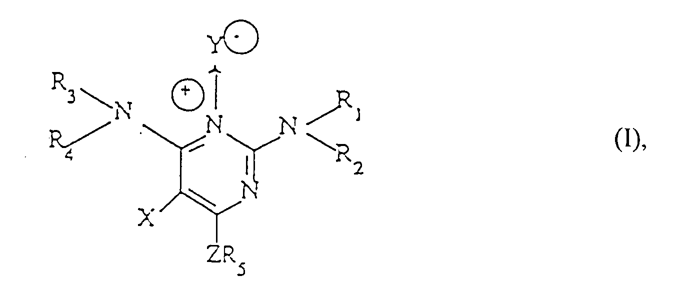 worin bedeuten:
R₁ und R₃ Wasserstoff;
R₂ und R₄, die identisch oder voneinander verschieden sind, Wasserstoff oder C₁₋₄-Alkyl;
R₅ Wasserstoff, C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist;
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie den Additionssalzen dieser Verbindungen mit kosmetisch und pharmazeutisch akzeptablen Säuren, mit der Maßgabe, daß 2,4-Diamino-6-hydroxy-5-brompyrimidin-3-oxid und 2,4-Diamino-6-thiophenylpyrimidin-3-oxid und deren Additionssalze mit einer Säure ausgenommen sind, dadurch gekennzeichnet, daß:
A) Zur Herstellung der Verbindungen der Formel (I), worin X Wasserstoff und Y Sauerstoff bedeutet, eine Verbindung der Formel (II): 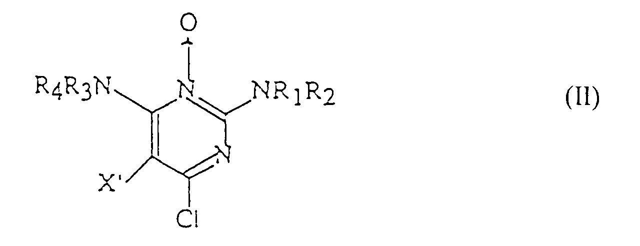 worin bedeuten:
R₁ und R₃ Wasserstoff;
R₂ und R₄ Wasserstoff oder C₁₋₄-Alkyl; und
X' Wasserstoff;
mit einem Alkoholat R₅O⁻ W⁺ in dem Alkohol R₅OH, worin R₅ die oben angegebene Bedeutung aufweist und W ein Alkalimetall bedeutet, oder mit einem Thiolat R₅S⁻ W⁺ in Gegenwart von Dimethylformamid umgesetzt wird;
B) zur Herstellung von Verbindungen der Formel (I), worin X Halogen und Y Sauerstoff bedeutet, die Verbindungen der oben definierten Formel (II) in Gegenwart eines Alkohols mit einem Halogenierungsmittel umgesetzt werden;
F) zur Herstellung von Verbindungen der Formel (I), worin Y OSO₃⁻ bedeutet, die Verbindungen der Formel (I), worin Y Sauerstoff bedeutet, mit einem Addukt von Schwefeltrioxid an Pydridin, Schwefeltrioxid anTriethylamin oder Schwefeltrioxid an Ethyldiisopropylamin in Gegenwart eines Lösungsmittels 1 bis 24 h bei 0 bis 25 °C sulfatiert werden.

17. Verwendung der Verbindungen der Formel (I) nach Anspruch 1 zur Herstellung eines Arzneimittels, das zur Behandlung von Alopezie, Pelade, Haarausfall oder desquamativer Dermatitis verwendet werden soll.

18. Verfahren zur kosmetischen Behandlung der Haare und der Kopfhaut, dadurch gekennzeichnet, daß eine Zusammensetzung nach einem der Ansprüche 1 bis 14 aufgetragen wird.

19. Verfahren zur Herstellung einer Zusammensetzung, um den Haarausfall zu verlangsamen und das Haarwachstum zu stimulieren, dadurch gekennzeichnet, daß in ein physiologisch akzeptables Medium mindestens eine Verbindung der folgenden Formel eingebracht wird: 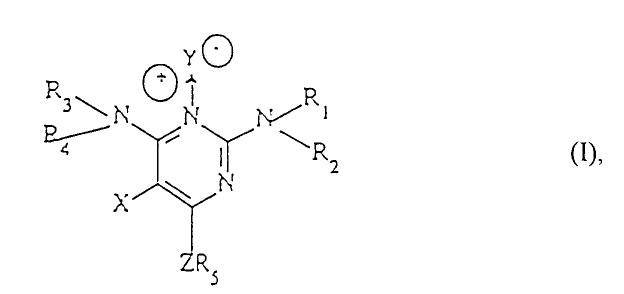 worin bedeuten:
R₁ und R₃ Wasserstoff;
R₂ und R₄, die identisch oder voneinander verschieden sind, Wasserstoff oder C₁₋₄-Alkyl,
R₅ Wasserstoff C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist;
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie die Additionssalze dieser Verbindungen mit physiologisch akzeptablen Säuren, mit der Maßgabe, daß die Verbindung der Formel (I) von 2,4-Diamino-6-hydroxy-5-brompyrimidin-3-oxid und 2,4-Diamino-6-thiophenylpyrimidin-3-oxid verschieden ist.

20. Zusammensetzung zur Verwendung als Arzneimittel, um den Haarausfall zu verlangsamen oder das Haarwachstum zu induzieren und zu stimulieren, dadurch gekennzeichnet, daß sie in einem physiologisch akzeptablen Medium mindestens eine Verbindung der folgenden Formel enthält: 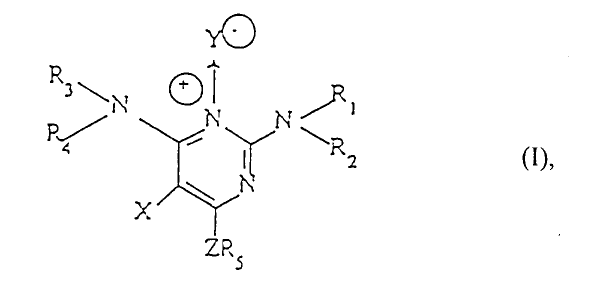 worin bedeuten:
R₁ und R₃ Wasserstoff;
R₂ und R₄, die identisch oder voneinander verschieden sind, Wasserstoff oder C₁₋₄-Alkyl,
R₅ Wasserstoff, C₁₋₁₂-Alkyl, C₃₋₁₂-Alkenyl, C₃₋₈-Cycloalkyl, Aryl und vorzugsweise Phenyl oder Tolyl, Arylalkyl und vorzugsweise Benzyl, Hydroxyalkyl oder Aminoalkyl, worin die Alkylgruppe 1 bis 6 Kohlenstoffatome aufweist;
X Wasserstoff oder Halogen;
Z Schwefel oder Sauerstoff; mit der Maßgabe, daß Z Schwefel bedeutet, wenn
X Wasserstoff bedeutet oder R₅ eine Arylgruppe ist;
Y Sauerstoff oder OSO₃;
sowie die Additionssalze dieser Verbindungen mit physiologisch akzeptablen Säuren.
